# EUROPÄISCHE PATENTSCHRIFT

(11) **EP 1 812 560 B1**
(45) Veröffentlichungstag und Bekanntmachung des Hinweises auf die Patenterteilung: **23.12.2009**
(21) Anmeldenummer: 05798063.3
(22) Anmeldetag: 26.10.2005
(51) Int. Cl.: C12N 5/06, C12N 5/08, A61K 35/28, A61K 48/00, A61P 27/02

(54) **IN VITRO AUS KNOCHENMARKSTAMMZELLEN DIFFERENZIERTE RETINA-SPEZIFISCHE ZELLEN, IHRE HERSTELLUNG UND VERWENDUNG**
RETINA-SPECIFIC CELLS DIFFERENTIATED IN VITRO FROM BONE MARROW STEM CELLS, THE PRODUCTION THEREOF AND THEIR USE
CELLULES SPECIFIQUES DE LA RETINE, DIFFERENCIEES IN VITRO A PARTIR DE CELLULES SOUCHES DE LA MOELLE OSSEUSE, LEUR PRODUCTION ET LEUR UTILISATION

(30) Priorität: 16.11.2004 DE 102004055615
(43) Veröffentlichungstag der Anmeldung: 01.08.2007
(73) Patentinhaber: Universitätsklinikum Hamburg-Eppendorf, 20246 Hamburg (DE)
(72) Erfinder: ZANDER, Axel, R., 22359 Hamburg (DE); ENGELMANN, Katrin, 01326 Dresden (DE); VALTINK, Monika, 01309 Dresden (DE); LANGE, Claudia, 22529 Hamburg (DE); FEHSE, Boris, 22527 Hamburg (DE)
(74) Vertreter: UEXKÜLL & STOLBERG
(86) Internationale Anmeldenummer: PCT/EP2005/011468
(87) Internationale Veröffentlichungsnummer: WO 2006/053629

(56) Entgegenhaltungen:
- WO-A-2004/010959
- US-A- 6 117 675
- TANG L ET AL: "Differentiation of Adult Stem Cells into retinal cells in vitro and in vivo" IOVS, Bd. 45, Nr. Suppl. 2, April 2004 (2004-04), Seite U649, XP009058479 & ANNUAL MEETING OF THE ASSOCIATION-FOR-RESEARCH-IN-VISION-AND-OPH THALM OLOGY; FT LAUDERDALE, FL, USA; APRIL 24 -29, 2004 ISSN: 0146-0404
- KICIC ANTHONY ET AL: "Differentiation of marrow stromal cells into photoreceptors in the rat eye." JOURNAL OF NEUROSCIENCE, Bd. 23, Nr. 21, 27. August 2003 (2003-08-27), Seiten 7742-7749, XP002358285 ISSN: 0270-6474
- RICHEL A ET AL: "Immunhistochemical study on human retinal pigment epithelia (HRPE) cells" IOVS, Bd. 40, Nr. 4, 15. März 1999 (1999-03-15), Seite S219, XP009058502 & ANNUAL MEETING OF THE ASSOCIATION FOR RESEARCH IN VISION AND OPHTHALMOLOGY; FORT LAUDERDALE, FLORIDA, USA; MAY 9-14, 1999
- VALTINK MONIKA ET AL: "Physiological features of primary cultures and subcultures of human retinal pigment epithelial cells before and after cryopreservation for cell transplantation" GRAEFE'S ARCHIVE FOR CLINICAL AND EXPERIMENTAL OPHTHALMOLOGY, Bd. 237, Nr. 12, Dezember 1999 (1999-12), Seiten 1001-1006, XP002358286 ISSN: 0721-832X
- ENGELMANN KATRIN ET AL: "RPE cell cultivation." GRAEFE'S ARCHIVE FOR CLINICAL AND EXPERIMENTAL OPHTHALMOLOGY = ALBRECHT VON GRAEFES ARCHIV FUR KLINISCHE UND EXPERIMENTELLE OPHTHALMOLOGIE. JAN 2004, Bd. 242, Nr. 1, Januar 2004 (2004-01), Seiten 65-67, XP002358287 ISSN: 0721-832X
- BARTSCH U ET AL: "Stem cell therapy in opthalmology" MEDIZINISCHE GENETIK 2003 GERMANY, Bd. 15, Nr. 2, 2003, Seiten 167-172, XP009058498 ISSN: 0936-5931

## Beschreibung

Die Erfindung betrifft Retina-spezifische Zellen, die aus menschlichen adulten Knochenmarkstammzellen abgeleitet sind, sowie auf deren Herstellung und Verwendung zur Herstellung einer pharmazeutischen Zusammensetzung zur Behandlung von Krankheiten, die mit erworbener oder angeborener Dysfunktion des retinalen Pigmentepithels der Retina oder der Chorioidea verbunden sind.

Degenerative Erkrankungen der Netzhaut (Retina) sind eine der Hauptursachen, die zu einem Verlust des Augenlichts führen. Solche Erkrankungen beruhen häufig auf Erkrankungen des retinalen Pigmentepithels (RPE).

Die Zellen des RPE variieren in der Größe von 10 bis 60 µm mit kleineren Zellen in der Fovea, die durch mehr und größere Melanosomen stark pigmentiert sind, und größeren und weniger stark pigmentierten Zellen mit weniger Melanosomen an der peripheren Retina. RPE-Zellen sind polarisiert mit einem villösen Apex auf der den Photorezeptoren zugewandten apikalen Seite und einer basalen Seite mit wenigen Einfaltungen. Die apikale Seite weist Mikrovilli auf, welche die Photorezeptor-Außensegmente umschließen. Die basale Seite ist der Bruchmembran zugewandt, auf der die Zellen aufsitzen bzw. an der sie "verankert" sind. RPE-Zellen gehören weiterhin zu den metabolisch aktivsten Zellen im Körper und enthalten zahlreiche Mitochondrien, rauhes Endoplasmatisches Reticulum, Golgi-Apparat und einen großen runden Kern. Gelegentlich kann eine Zelle 2 Kerne enthalten. Mit dem Alter nimmt die Zahl der Zellen mit zwei Kernen zu.

Die Rolle der RPE-Zellen ist vielgestaltig und umfaßt verschiedene Aufgaben
➢ im Vitamin A-Metabolismus (z.B. bei der Aufnahme von Vitamin A aus dem Blutstrom und dessen Umwandlung in 11-cis-Retinal, welches an die Photorezeptoren abgegeben wird, dort an Opsin bindet und dadurch Rhodopsin bildet; dieses wird während des Sehvorgangs durch Licht deaktiviert, dadurch verbraucht und in der verbrauchten Form zum RPE zurück transportiert, wo es erneut zu 11-cis-Retinal umgesetzt wird);
➢ als äußere Blut-Retina-Barriere;
➢ in der Phagozytose von äußeren Segmenten der Photorezeptoren, da jede RPE-Zelle bis zu 4000 Scheiben der äußeren Segmente der Photorezeptoren pro Tag aufnehmen kann, die in Phagosomen eingekapselt und nachfolgend in Lysosomen abgebaut werden;
➢ in der Absorption von Licht durch Absorption des Streulichts, wodurch dieses minimiert wird;
➢ in der Bildung der Interphotorezeptormatrix, die z.B. an der Haftung der Retina an das RPE beteiligt ist;
➢ in dem aktiven Transport von Wasser und Metaboliten wie z.B. D-Glukose und Tyrosin über Na⁺/K⁺-ATPase-Pumpen auf der apikalen Oberfläche und Chlorid-Bicarbonat-Transportern auf der basalen Oberfläche;
➢ in der Reaktion auf mechanische und thermale Schädigung durch Reparatur, Regenerierung, fibrovaskuläre Proliferation und Pigmentmigration; und
➢ beim Abfangen von Toxinen und Radikalen durch Bindung an das im RPE gelegene Melanin, wodurch die Chorioidea und die Retina als benachbarte Strukturen vor oxidativer Schädigung geschützt werden.

Wegen ihrer herausragenden, das Sehen ermöglichenden Rolle im Auge führen erworbene oder angeborene Dysfunktionen der RPE-Zellen, d.h. Verlust der Zellintegrität, Proliferation oder Migration der Zellen mit der sekundären Konsequenz einer Degenerierung der nicht-regenerierenden Photorezeptoren unausweichlich zu konsekutivem, irreversiblem Verlust des (zentralen) Sehens.

Zusätzlich kann die basal an das RPE grenzende Choriokapillaris (Lamina choroidocapillaris) als Folge einer Degenerierung des RPE ebenfalls degenerieren, was eine pathologische Neovaskularisation zur Folge hat. Diese Pathologie wird von Blutungen aus den neuen Gefäßen begleitet und führt zu einer weiteren Verschlechterung des Sehvermögens [HOLZ, F.G. & PAULEIKHOFF, D. (1996) Ophthalmologe 93: 299-315].

Eine derartige Degeneration der Chorioidea (Aderhaut) tritt häufig im Verlauf einer Diabeteserkrankung auf.

Ein Beispiel für eine erworbene retinale Erkrankung, die ihre Ursache im RPE hat, ist die altersabhängige Makuladegeneration (AMD), unter der etwa 20% der Patienten über 65 leiden [WILLIAMS, R.A. et al. (1998) Arch Ophthalmol 116: 514-520; YOUNG, R.W. (1987) Surv Ophthalmol 31: 291-306]. Makuladegeneration ist die ungenaue historische Bezeichnung einer Gruppe von Krankheiten, welche in den licht-sensorischen Zellen in der Makulazone der Retina zu Fehlfunktion oder Funktionsverlusten führen und letztlich in einem schwächenden Verlust der vitalen zentralen oder peripheren Sicht führen. Bis heute konnte die Pathogenese von AMD nicht ausreichend geklärt werden [HOGAN, M.J. (1972) Trans Am Acad Ophthalmol Otol 76: 64-80; YOUNG, R.W. (1987) Surv Ophthalmol 31: 291-306; LAHIRI-MUNIR, D. (1995) "Retinal Pigment Epithelial Transformation." Springer-Verlag, Heidelberg].

Ein Beispiel einer angeborenen Degenerierung der Retina ist die Retinopathia pigmentosa, bei der es sich um eine Gruppe von Erkrankungen handelt, die auch als Retinitis pigmentosa bezeichnet werden und durch eine Degenerierung des Retinalepithels ohne begleitende Entzündung, durch Atrophierung des optischen Nervs und weitläufige Pigmentveränderungen in der Retina gekennzeichnet sind, die in einer fortschreitenden Abnahme der Sicht münden. Retinitis pigmentosa mit ihren zahlreichen Unterformen ist eine der häufigsten Gründe für Blindheit gerade bei Menschen ab dem 30. Lebensjahr [vgl. LORENZ, B. et al. (2001) Dt. Ärztebl 98: A3445-3451; Information der Patientenvereinigung "Pro Retina e.V." unter www.pro-retina.de].

Therapeutische Ansätze für eine Behandlung oder Heilung von retinalen Erkrankungen, die heutzutage angewandt werden, einschließlich Lasertherapie oder chirurgische Entfernung von Neovaskularisationsmembranen setzen relativ spät im Krankheitsverlauf ein und sind im besten Fall nur in der Lage, die Krankheit aufzuhalten. Bis heute gibt es keine Heilung für retinale Erkrankungen.

Die Verwendung von voll funktionalen Spenderzellen (RPE) als Transplantat zum Ersatz für erkrankte Zellen bietet einen vielversprechenden Ansatz in Richtung Heilung solcher Krankheiten. Gewöhnlich werden Spenderzellen postmortal aus Spenderaugen entnommen und entweder frisch oder nach einem Kulturschritt verwendet. Nachteile der postmortal entnommenen Zellen sind eine verringerte Vitalität und ein durch den Kultivierungsschritt verschlechterter Differenzierungsstatus der Zellen. Trotz dieser Nachteile ist es gelungen, Transplantationen mit mittelfristigem Erfolg im Tiermodell zu erreichen [vgl. ALGERVE, P.V. et al. (1997) Graefe's Arch Clin Ophthalmol 235: 149-158; CRAFOORD, S. et al. (1999) Acta Ophthalmol Scand 77: 247-254; GOURAS, P. et al. (1985) Curr Eye Res 4: 253-265; GOURAS, P. et al. (1989) Prog Clin Biol Res 314: 659-671; LI, L. et al. (1988) Exp Eye Res 47: 771-785; LI, L. et al. (1991) Exp Eye Res 52: 669-679; LITTLE, C.W. et al. (1996) Invest Ophthalmol Vis Sci 37: 204-211; PEYMAN, G.A. et al. (1991) Ophthal Surg 22: 102-108; SHEEDLO, H.J. et al. (1989) Exp Eye Res. 48: 841-854; SEILER, M.J. & ARAMANT, R.B. (1998) Invest Ophthalmol Vis Sci 39: 2121-2131]. Versuche einer Transplantation bei Humanpatienten scheiterten jedoch an der geringen Qualität der Spenderzellen. Bislang wurden andere retinale Zellen, wie z.B. Photorezeptoren, nur experimentell und nur als embryonale Zelle verpflanzt [vgl. ARAMANT, R.B. et al. (1999) Invest Ophthalmol Vis Sci 40: 1557-1564], wodurch dieser Ansatz zur Zeit nach heutigen wissenschaftlichen und ethischen Standards für eine Therapie nicht denkbar ist.

TANG L ET AL: "Differentiation of Adult Stem Cells into retinal cells in vitro and in vivo" IOVS, Bd. 45, Nr. Suppl. 2, April 2004 (2004-04), Seite U649, XP009058479 & ANNUAL MEETING OF THE ASSOCIATION-FOR-RESEARCH-IN-VISION-AND-OPH THALM OLOGY; FT LAUDERDALE, FL, USA; APRIL 24 -29, 2004 und KICIC ANTHONY ET AL: "Differentiation of marrow stromal cells into photoreceptors in the rat eye." JOURNAL OF NEUROSCIENCE, Bd. 23, Nr. 21, 27. August 2003 (2003-08-27), Seiten 7742-7749 beschreiben eine Differenzierung von knochenmarksstammzellen in Retina-spezifische Zellen in verschiedenen Medien.

In Anbetracht der ungelösten Probleme besteht die der Erfindung zugrunde liegende Aufgabe in der Bereitstellung einer Therapie für retinale Pathologien.

Entsprechend der vorliegenden Erfindung wird dieses Problem durch Differenzierung von mesenchymalen oder hämatopoetischen Stammzellen aus dem Knochenmark oder einer Mischung aus beiden Zelltypen in Retina-spezifische Zellen gelöst, insbesondere durch Verfahren nach den Ansprüchen 1 bis 29 und 43, eine Verwendung nach einem der Ansprüche 30 bis 38 und 50 bis 53, Zellen bzw. Zellzubereitungen nach den Ansprüchen 39 bis 42 bzw. 44 bis 47 und/oder eine pharmazeutische Zubereitung nach Anspruch 49.

### Beschreibung der Figuren

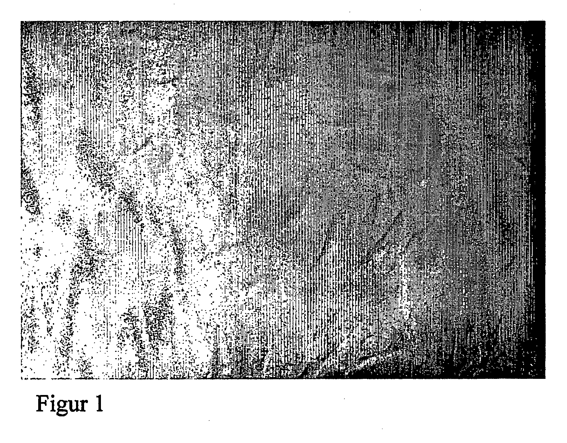

- Figur 1:: Lichtmikroskopische Aufnahme von isolierten, undifferenzierten mesenchymalen Stammzellen nach 2 Tagen in Kultur in Anwesenheit von CCM als Differenzierungsmedium (Vergrößerung × 100).

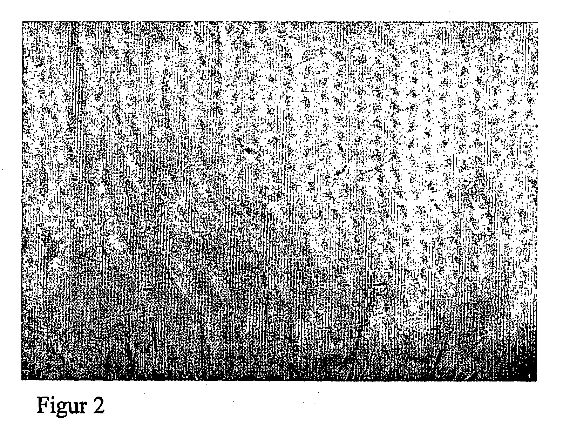

- Figur 2:: Lichtmikroskopische Aufnahme von isolierten mesenchymalen Stammzellen nach 5 Tagen in Kultur in Anwesenheit von CCM als Differenzierungsmedium mit beginnender Differenzierung der Stammzellen in Zellen mit neural erscheinender Zellmorphologie, die durch Ausbildung dendritischer Fortsätze gekennzeichnet sind (Vergrößerung × 100).

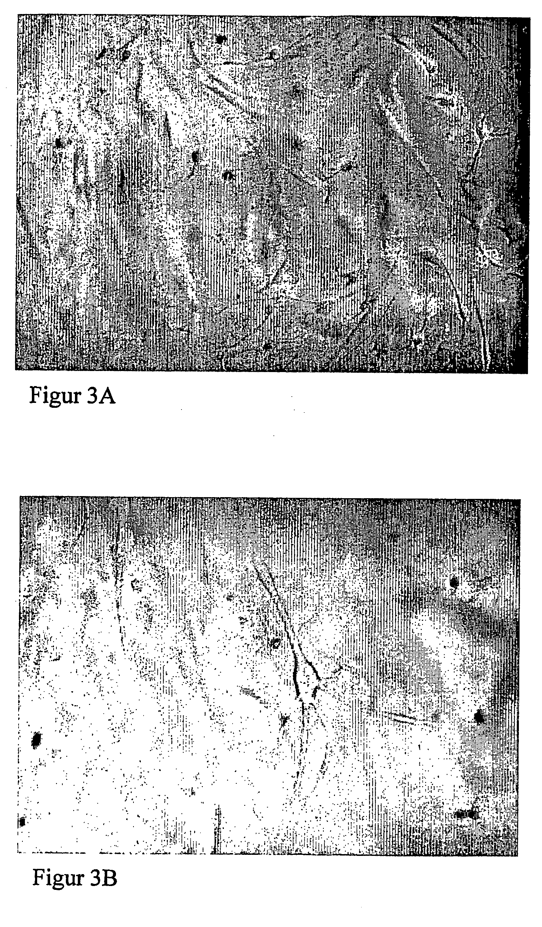

- Figur 3:: Lichtmikroskopische Aufnahme von isolierten mesenchymalen Stammzellen nach 9 Tagen in Kultur in Anwesenheit von CCM als Differenzierungsmedium mit weiter fortgeschrittener Differenzierung der Stammzellen in Zellen mit neural erscheinender Zellmorphologie, die durch beginnende Verästelung der dendritischen Fortsätze gekennzeichnet sind (Vergrößerung in Figur 3A × 100 und Figur 3B × 200).

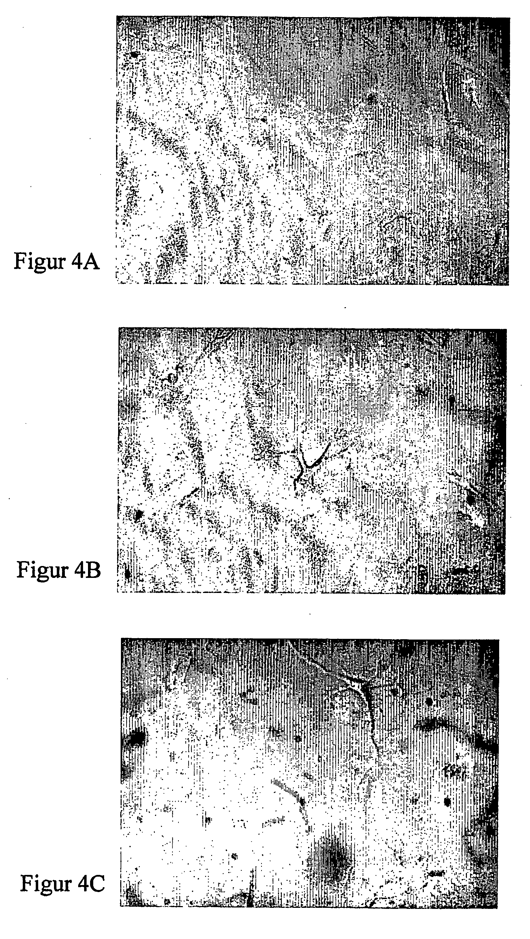

- Figur 4:: Lichtmikroskopische Aufnahme von isolierten mesenchymalen Stammzellen, die nach 14 Tagen Kultur in Anwesenheit von CCM als Differenzierungsmedium eine neurale Zellmorphologie mit dendritischen Fortsätzen und Verästelungen aufweisen (Vergrößerung in Figur 4A × 100, Figur 4B × 200 und Figur 4C × 320).

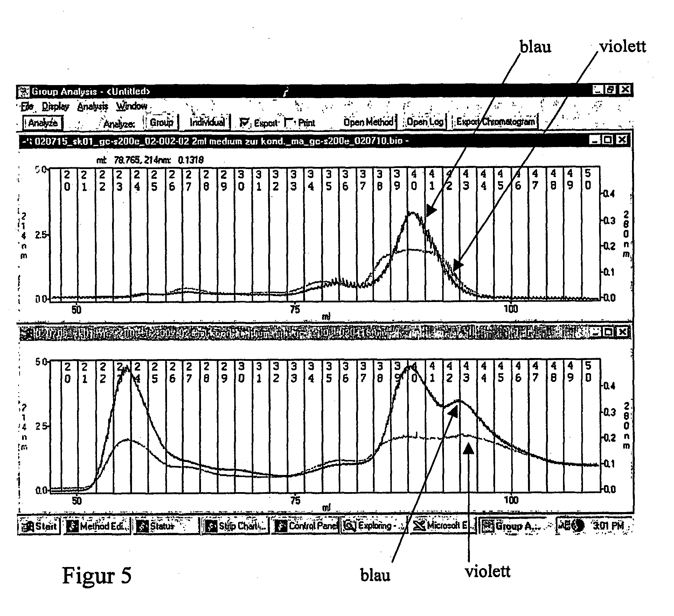

- Figur 5:: Chromatogramm (violette Kurve - 214 nm; blaue Kurve - 280 nm) der Fraktionen 20 - 50 eines Kulturmediums vor Inkubation von Chorioidea in diesem Medium (oberer Teil der Figur) und eines konditionierten Mediums, das nach Inkubation von Chorioidea in diesem Medium erhalten wird (unterer Teil der Figur); Darstellung der Veränderungen der Proteinzusammensetzung.

Der Begriff "Retina-spezifische Zellen, wie er hier verwendet wird, bezeichnet eine Untergruppe neuraler Zellen, die natürlicherweise in der Retina vorkommen. Weiterhin werden von diesem Begriff Zellen mit neuraler Morphologie umfaßt, die spezifischen Zellen aus der Retina gleichen und deren Funktion(en) ausüben.

Der Begriff "Stammzellen", wie der hier verwendet wird, bezeichnet adulte mesenchymale oder hämatopoetische Stammzellen aus dem Knochenmark, die aus einem Knochenmarkaspirat durch geeignete, dem Fachmann bekannte Verfahren gewonnen werden können. Diese Verfahren zur Gewinnung von Knochenmark sind für den Spender unbedenklich und werden im Rahmen eines kleinen Eingriffs durchgeführt.

In einer bevorzugten Ausführungsform der Erfindung werden isolierte und expandierte Stammzellen aus dem Knochenmark mit einem Verfahren in Retina-spezifische Zellen differenziert, bei dem man
a) die Stammzellen in einem geeigneten Kulturmedium expandiert;
b) die expandierten Stammzellen in einem Differenzierungsmedium kultiviert; und
c) die Retina-spezifischen Zellen durch Trennung der Zellen vom Differenzierungsmedium isoliert.

In einer Ausführungsform der Erfindung werden dabei in diesem Differenzierungsverfahren adulte mesenchymale Stammzellen aus dem Knochenmark als Ausgangsmaterial verwendet.

Überraschenderweise konnte gezeigt werden, daß das erfindungsgemäße Verfahren zu einer Differenzierung zu Retina-spezifischen Zellen führt.

Dabei konnte zum ersten Mal überhaupt gezeigt werden, daß mesenchymale Stammzellen mit ihrer bekannten Fähigkeit einer Differenzierung in eine große Zahl verschiedener Zellen, wie z.B. Knochen, Knorpel, Lunge, Milz, Zentralnervensystem, Muskeln und Leberzellen [vgl. PEREIRA, R.F. et al. (1995) Proc Natl Acad Sci USA 92: 4857-4861; AZIZI, S. et al. (1998) Proc Natl Acad Sci USA 95: 3908; FERRARI, G. et al. (1998) Science 279: 1528-1530; KOPEN, G.C. et al. (1999) Proc Natl Acad Sci USA 96: 10711-10716], in vitro auch in Retina-spezifische Zellen differenziert werden können.

Die erfindungsgemäß verwendeten mesenchymalen Stammzellen exprimieren mindestens zwei typische Oberflächenantigene ausgewählt aus der Gruppe bestehend aus CD59, CD90, CD105 und MHC I. Die erfindungsgemäßen mesenchymalen Stammzellen sind jedoch nicht allein durch die Expression eines oder mehrerer spezifischer Oberflächenmarker gekennzeichnet, sondern generell durch das Expressionsmuster einer Vielzahl von Antigenen, das sich durch die Nachweisbarkeit (Expression vorhanden) bzw. die fehlende Nachweisbarkeit (keine Expression vorhanden) dieser Antigene in spezifischen Nachweisverfahren auszeichnet. So ist z.B. keine Expression von CD34 und CD45 messbar. In einer besonders bevorzugten Ausführungsform exprimieren die mesenchymalen Stammzellen die Oberflächenantigene CD105 (Endoglin) und CD90 (Thy-1).

Die Expression dieser spezifischen Marker (Oberflächenantigene) kann durch kommerziell erhältliche Antikörper mit Spezifität gegen die jeweiligen Antigene unter Verwendung von Standard-Immunnachweisverfahren detektiert werden [vgl. LOTTSPEICH F. & ZORBAS H. "Bioanalytik", Spektrum Akademischer Verlag GmbH, Heidelberg-Berlin (1998))}]. Der Nachweis des Gesamt-MHC I-Komplex erfolgt beispielsweise mit dem Antikörper gegen HLA-A,B,C (Hersteller BD Pharmingen, Katalognummer 555552).

Während der Proliferations- oder Vermehrungsphase der Zellen haftet eine unterschiedliche Anzahl der Zellen am Boden oder an der Wand des jeweiligen Kulturgefäßes an. Für die Differenzierung in die Retina-spezifischen Zellen in Stufe b) des erfindungsgemäßen Verfahrens werden die adhärent wachsenden, expandierten mesenchymalen Stammzellen verwendet (vgl. Beispiel 2).

In einer weiteren Ausführungsform der Erfindung werden in dem erfindungsgemäßen Differenzierungsverfahren adulte hämatopoetische Stammzellen aus dem Knochenmark als Ausgangsmaterial verwendet.

Die erfindungsgemäß verwendeten hämatopoetischen Stammzellen exprimieren mindestens ein typisches Oberflächenantigen ausgewählt aus der Gruppe bestehend aus CD34 und CD45. Analog zu den erfindungsgemäßen mesenchymalen Stammzellen sind die erfindungsgemäßen hämatopoetischen Stammzellen ebenfalls nicht allein durch die Expression eines oder mehrerer spezifischer Oberflächenmarker gekennzeichnet, sondern generell durch das Expressionsmuster einer Vielzahl von Antigenen. In einer besonders bevorzugten Ausführungsform exprimieren die hämatopoetischen Stammzellen die Oberflächenantigene CD34 und CD45.

Der Nachweis einer Expression der spezifischen Marker (Oberflächenantigene) für die hämatopoetischen Stammzellen kann ebenfalls durch kommerziell erhältliche spezifische Antikörper durch Verwendung von Standard-Immunnachweisverfahren erfolgen [vgl. LOTTSPEICH F. & ZORBAS H. "Bioanalytik", Spektrum Akademischer Verlag GmbH, Heidelberg-Berlin (1998)}]

Die Aufreinigung der erfindungsgemäßen hämatopoetischen Stammzellen kann mittels MACS ("Magnetic-activated Cell Sorting"; Firma Miltenyi) erfolgen. Bei dieser Technik erfolgt die Aufreinigung über innerhalb eines Magneten platzierte Säulen, auf die Zellen des Knochenmarks gegeben werden, welche mit Antikörpern inkubiert wurden, die mit Ferromagneten gekoppelt sind. Komplexe aus Stammzellen und Antikörpern binden an die Säule und können so spezifisch aufgereinigt werden [SUTHERLAND, et al. (1996) J Hematotherapy 5: 213-226]. Weitere Verfahren sind dem Fachmann geläufig.

Für die Differenzierung in die Retina-spezifischen Zellen in Stufe b) des erfindungsgemäßen Verfahrens werden die hämatopoetischen Stammzellen bevorzugt unmittelbar nach ihrer Aufreinigung verwendet. Eine weitere Kultur bzw. Expansion der aufgereinigten Zellen ist jedoch auch erfindungsgemäß umfasst.

In einer weiteren Ausführungsform der Erfindung wiederum werden in dem erfindungsgemäßen Differenzierungsverfahren Stammzellen aus dem Knochenmark aus Ausgangsmaterial verwendet, die sowohl mesenchymale als auch hämatopoetische Stammzellen umfassen. Dabei ist sowohl die direkte Verwendung des aus dem Knochenmark entnommenen Aspirats als auch jegliche Mischung erfindungsgemäß umfasst, welche die zuvor isolierten mesenchymalen sowie die zuvor isolierten hämatopoetischen Stammzellen nachträglich wieder vereint enthält.

Nach Erhalt der Retina-spezifischen Zellen in Stufe c) des Differenzierungsverfahrens werden diese vorzugsweise in einem geeignetem Zellkulturmedium suspendiert und nachfolgend für eine Lagerung ohne Verlust ihres therapeutischen Potentials tiefgefroren. Bevorzugt ist dieses Medium ein Standardmedium ausgewählt aus der Gruppe bestehend aus RPMI, Medium 199, DMEM (low glucose; dieses Medium entspricht dem Modified Eagles's Medium (Gibco 31885) und Iscove's Medium jeweils allein oder als 1:1-Mischung mit Ham's F12 Nutrient Mixture. Das Medium kann ferner ein Spezialmedium sein ausgewählt aus der Gruppe bestehend aus Medium Human Endothelial-SFM (Gibco 11111), START V (Biochrom F8075) und Neurobasal- bzw. Neurobasal-A Medium (Gibco 21103 bzw. 10888) sowie deren Supplemente N-2 (Gibco 17502) oder B27 (Gibco 17504-044). Diese Medien werden mit oder ohne Zusatz eingesetzt. Ein möglicher Zusatz für die genannten Spezialmedien ist Ham's F12 Nutrient Mixture, das einen hohen Gehalt an Aminosäuren und Vitaminen aufweist. Dem ausgewählten Medium werden bevorzugt DMSO oder Methylzellulose als Gefrierschutz und Proteine zur Stabilisierung empfindlicher biologischer Substanzen zugesetzt.

Besonders bevorzugt werden als Gefrierschutz 10 % DMSO und zur Stabilisierung empfindlicher biologischer Substanzen mindestens 10 % Serum (bzw. Albumin bei serumfreier Kultur) zugesetzt.

Das Medium DMEM (low glucose) kann wahlweise mit HEPES (Gibco 22320) als zusätzlicher Puffersubstanz oder ohne diesen Zusatz verwendet werden. HEPES als Puffersubstanz stabilisiert den pH des Mediums effektiver als z.B. ein Carbonat- oder Phosphat-Puffer und wird von den Stammzellen gut vertragen.

Bei der erfindungsgemäßen Verwendung von Neurobasal bzw. Neurobasal-A Medium ist zu beachten, daß diese Medien bevorzugt nur für die Kultur der in der Stufe c) des erfindungsgemäßen Verfahrens erhaltenen differenzierten Zellen verwendet werden, da die Überlebensfähigkeit undifferenzierter Stammzellen in diesen Medien drastisch reduziert ist.

Die in vitro-Differenzierung der erfindungsgemäßen Retina-spezifischen Zellen bzw. die Einleitung der Differenzierung der Zellen (das "priming"), die morphologisch nicht sichtbar ist und sich erst nach Transplantation der Zellen ins Auge unter dem Einfluß der umliegenden Gewebe vollendet, erfolgt auf einfache und zuverlässige Weise, indem man die Zellen in Schritt b) in einem besonderem Medium kultiviert. Dieses Medium enthält entweder den Überstand eines Kulturmediums, in dem Chorioideae und/oder Teile davon kultiviert wurden (vgl. Beispiel 3), oder den Überstand, den man nach abgeschlossenem Homogenisieren von Retina durch Zentrifugation erhält (siehe Beispiel 4). Dieses Medium wird nachfolgend allgemein als "Differenzierungsmedium" bezeichnet.

Vorzugsweise enthält das Differenzierungsmedium Chorioidea-konditioniertes Medium (CCM) oder Retinaextrakt (RE) [vgl. PFEFFER, B.A. (1991) Prog Retina Res 10: 251-291; Hu, J. & BOK, D. (2001) Mol Vis 7: 14-19; VENTURA, A.C. et al. (1996) Ophthalmologie 93: 262-267; VALTINK, M. et al. (1999) Graefe's Arch Clin Exp Ophthalmol 237: 1001-1006; COULOMBE, J.N. et al. (1993) Neuron 10: 899-906]; ENGELMANN, K. et al. (2004) Graefe's Arch Clin Exp Ophtalmol 242:65 - 67.

Gemäß einer besonderen Ausführungsform kann CCM auch in Verbindung mit RE eingesetzt werden.

Für die Differenzierung der Stammzellen in Retina-spezifische Zellen kann zur Gewinnung des Chorioidea-konditionierten Mediums ein Verfahren angewendet werden, bei dem man
a) das anteriore Segment, den Glaskörper und die neurosensorische Retina aus humanen Spenderaugen entfernt;
b) die Chorioidea und/oder Fragmente davon vom Auge abpräpariert;
c) anhaftende, zum retinalen Pigmentepithel gehörende Zellen von der präparierten Chorioidea und/oder den Fragmenten davon durch Waschen und nachfolgende Inkubation in einer Kollagenaselösung entfernt;
d) die Chorioidea und/oder Fragmente davon in einem geeigneten Kulturmedium inkubiert; und
e) den Überstand des Kulturmediums nach erfolgter Inkubation als Differenzierungsmedium sammelt.

Standardzellkulturmedien können als Kulturmedium in Schritt c) dieses Verfahrens verwendet werden (vgl. Beispiele). Die Kultur der Chorioideae erfolgt bei 37°C in feuchter Atmosphäre (90 bis 97 % Luftfeuchtigkeit) in einem Inkubator in einem Gasgemisch aus 5 % CO₂ und 95 % Luft.

In bevorzugten Ausführungsformen der Erfindung werden als Kulturmedien zur Herstellung des Chorioidea-konditionierten Mediums Standardmedien wie RPMI, Medium 199, DMEM (low glucose; entspricht dem Modified Eagle's Medium (Gibco 31885)) oder Iscove's Medium jeweils allein oder in einer 1:1-Mischung mit Ham's F12 Nutrient Mixture verwendet. DMEM (low glucose) kann wahlweise mit HEPES (Gibco 22320) als zusätzlicher Puffersubstanz oder ohne diesen Zusatz verwendet werden. Ferner enthält das Kulturmedium als weiteren Zusatz fötales Kälberserum (FCS).

Bevorzugt wird zur Herstellung des Chorioidea-konditionierten Mediums eine 1:1-Mischung aus Medium 199 und Ham's F12 verwendet, das mit 1% (v/v) FCS supplementiert ist.

Weiterhin kann in dem Medium zur Herstellung des Chorioidea-konditionierten Mediums das Serum durch Serumersatzstoffe ersetzt werden. Diese Serumersatzstoffe sind bevorzugt ausgewählt aus der Gruppe bestehend aus Insulin, Albumin (Gibco 11020 oder 11021), Transferrin, Selen und weiteren Spurenelementen, Lipiden, Lipoproteinen, Ethanolamin/Phosphoethanolamin und weiteren Hormonen wie Hydrocortison.

Die Serumersatzstoffe Insulin, Transferrin und Selen werden erfindungsgemäß bevorzugt als ITS-Supplement (Gibco 51300) eingesetzt. Bei Verwendung der einzelnen Substanzen beträgt der bevorzugte Konzentrationsbereich der einzelnen Substanzen beim Insulin 1 - 10 µg/ml, beim Transferrin 1 - 20 µg/ml und 20 nM beim Selen.

Die Spurenelemente sind bevorzugt ausgewählt aus der Gruppe bestehend aus Mangan, Zinn, Nickel, Vanadium oder Molybdän. Lipide und Lipoproteine werden bevorzugt als fertiges, für den Zellkulturbereich optimiertes Supplement eingesetzt (z.B. Sigma F7175, L0288, L9655 oder L0163).

Ethanolamin oder Phosphoethanolamin werden dem Medium zugesetzt, da sie von den Zellen sowohl zur Unterstützung des Lipidtransports als auch in serumfreien Medien bzw. Medien ohne Serumsupplementation essentiell bei der Phospholipidbiosynthese zum Aufbau der Zellmembran benötigt werden. Sie werden in der für Zellkulturen üblichen Standardkonzentration von bis zu 50 µmol/l eingesetzt [vgl. GRAFF, L. et al. (2002) Am J Pathol 160: 1561-1565; KIM, E.J. (1999) In Vitro Cell Dev Biol Anim 35(4): 178-182]. Hydrocortison wird bevorzugt in einer Konzentration von 1 - 10 nM eingesetzt und dient der Ernährung u.a. neuraler Zellen im Kulturmedium.

In einer weiteren bevorzugten Ausführungsform der Erfindung wird als Medium in Schritt c) zur Herstellung des Chorioidea-konditionierten Mediums durch Kultur der Chorioidea und/oder Fragmente davon ein synthetischer Serumersatz verwendet, der in fertiger Konzentration alle minimal erforderlichen Substanzen enthält. Besonders bevorzugt wird dabei der synthetische Serumersatz Biochrom K3611 oder K3620 verwendet.

In einer Ausführungsform der Erfindung werden die Chorioideae zur Herstellung des Chorioidea-konditionierten Mediums (CCM) über einen Zeitraum von 2 bis 8 Tagen, bevorzugt von 4 Tagen inkubiert.

Der Überstand dieser Kultur wird als konditioniertes Medium bevorzugt am Ende der Inkubation gewonnen. Es wird keine Inkubation mit zwischenzeitlichem Abnehmen des Überstandes zur Erzeugung einer größeren Menge konditionierten Mediums durch Vereinigung der einzelnen Überstände durchgeführt, da dieses mehrmalige "Melken" der Kultur zu einem Differenzierungsmedium mit schlechterer Qualität und z.T. inhibierender Wirkung führt.

In einer bevorzugten Ausführungsform der Erfindung werden die Chorioidea eines Spenderauges nach enzymatischer Ablösung der zum retinalen Pigmentepithel gehörenden Zellen 4 Tage in Medium F99, dem 1% (v/v) FCS zugegeben wurden, inkubiert. Nach Beendigung der Kultur wird das konditionierte Medium durch Zentrifugation gewonnen (vgl. Beispiele).

Zur Gewinnung des Retina-Extraktes (RE) zur Differenzierung der Stammzellen in Retina-spezifische Zellen kann ein Verfahren angewendet werden (siehe Beispiel 4), bei dem man
a) aus humanen Spenderaugen die Retina isoliert;
b) die Retina unter Zusatz von Proteinasehemmern homogenisiert; und
c) aus dem Homogenat durch Zentrifugation den Überstand als Retina-Extrakt gewinnt.

Das Chorioidea-konditionierte Medium (CCM) bzw. der Retinaextrakt (RE) als Zusatz für das Differenzierungsmedium werden jeweils unter sterilen Bedingungen filtriert und bei ca. - 20°C gelagert oder direkt für die Differenzierung der mesenchymalen Stammzellen eingesetzt (siehe Beispiele). Wie oben gezeigt, erfolgt die erfindungsgemäße Differenzierung der Stammzellen in Retina-spezifische Zellen bzw. die Induktion dieser Differenzierung durch Wachstum der Stammzellen in Gegenwart eines Differenzierungsmediums, das entweder den Überstand eines Kulturmediums, in dem Chorioideae und/oder Teile davon kultiviert wurden, oder den Überstand enthält, den man nach abgeschlossenem Homogenisieren von Retina durch Zentrifugation erhält (vgl. Beispiele 3 und 4).

In einer Ausführungsform der Erfindung werden die Stammzellen in Gegenwart von 1 bis 20 % CCM bzw. in Gegenwart von 0,1 bis 5,0 % RE inkubiert.

In einer bevorzugten Ausführungsform der Erfindung werden die Stammzellen in Gegenwart von 1 - 15 % CCM bzw. in Gegenwart von 0,5 - 5 % RE inkubiert.

In einer besonders bevorzugten Ausführungsform werden die Stammzellen in Gegenwart von 10 % CCM bzw. in Gegenwart von 1 % RE inkubiert.

In einer Ausführungsform der Erfindung werden die Stammzellen zur Differenzierung in Retina-spezifische Zellen für einen Zeitraum von 3 bis 21 Tagen in Gegenwart des Differenzierungsmediums kultiviert.

Bevorzugt werden die Stammzellen zur Differenzierung in Retina-spezifische Zellen für einen Zeitraum von 14 bis 21 Tagen in Gegenwart des Differenzierungsmediums kultiviert.

Nach 3 bis 5 Tagen treten durch die Differenzierung in Anwesenheit des Differenzierungsmediums erste morphologische Veränderungen an den Zellen auf, die zunächst eine stellare Morphologie annehmen. Diese Veränderung manifestiert sich im Laufe von bis zu 3 Wochen (vgl. Figuren 2 bis 4). Nach 14 Tagen in Kultur sind die Zellen ausdifferenziert, da keine weitere Differenzierung zu beobachten ist und die Zellen ihre veränderte, nun neuronal erscheinenden Morphologie beibehalten.

Die Zellen unterscheiden sich nach dem Differenzierungsschritt von un- bzw. ausdifferenzierten Zellen durch ihre Morphologie (vgl. Figuren 1 bis 4). Undifferenzierte Zellen sind länglich (siehe Figur 1), während Zellen nach Induktion der Differenzierung in Retina-spezifische Zellen Ausläufer ausbilden und eine sternförmige, stellare Form annehmen (siehe Figur 2). Einige dieser sternförmigen Zellen zeigen granuläre, dunkel erscheinende Ansammlungen um den Kern. Die Zellen verändern ihre Morphologie im weiteren Verlauf der Differenzierung und bilden dendritenartige Fortsätze und Verästelungen aus. Nach etwa 9 Tagen lassen sich erste neural erscheinende Zellen beobachten, während die stellaren Zellen langsam in der Kultur nicht mehr auffindbar waren.

Die neural erscheinenden Zellen weisen phänotypische Ähnlichkeit mit aus kultivierten neuralen Stammzellen differenzierten Astrozyten und Oligodendrozyten auf. Nach ca. 9 bis 14 Tagen treten an den Enden der verästelten Zellausläufer kleine Verdickungen auf, die morphologisch wie Podien aussehen (siehe Figuren 3 und 4). In der weiteren Entwicklung werden die bereits gebildeten Zellausläufer dicker, neue Zellausläufer werden kaum gebildet. Dieser Phänotyp bleibt in den folgenden 5 bis 7 Tagen erhalten.

In einer weiteren Ausführungsform werden die Stammzellen nur für einen kurzen Zeitraum von 3 bis 14 Tagen in Gegenwart des Differenzierungsmediums kultiviert, um die Differenzierung der Stammzellen in Retina-spezifische Zellen zu induzieren, wobei der sich an die Induktion anschließende Differenzierungsprozeß nicht vollendet wird.

Erfindungsgemäß besonders bevorzugt werden die Stammzellen zur Andifferenzierung in Retina-spezifische Zellen für einen Zeitraum von 3 bis 9 Tagen in Gegenwart des Differenzierungsmediums kultiviert.

Die Vollendung der Differenzierung der an- bzw. vordifferenzierten Zellen in Retina-spezifische Zellen erfolgt bei diesen Ausführungsbeispielen nach Verabreichung der Zellen ins Auge *in vivo* unter dem Einfluß des Mikroenvironments des Auges.

In weiteren Ausführungsformen werden die Stammzellen in einem mehrstufigen Verfahren differenziert, bei dem sowohl CCM als auch RE zur Differenzierung eingesetzt werden. Dabei folgt auf eine 3 bis 14 Tage dauernde Andifferenzierung der isolierten und expandierten Stammzellen in einem CCM enthaltenden Differenzierungsmedium eine bis zu 4 Wochen dauernde Kultur der Zellen in einem RE enthaltenden Differenzierungsmedium.

In einer besonderen Ausführungsform der Erfindung folgt dabei auf eine 3- bis 5-tägige Kultur in einem CCM enthaltenden Differenzierungsmedium eine Kultur der Zellen in einem RE enthaltenden Differenzierungsmedium für 1 bis 14 Tage, um z.B. retinales Pigmentepithel zu erhalten.

In einer weiteren besonderen Ausführungsform werden die Stammzellen nach der Kultur in einem CCM enthaltenden Differenzierungsmedium statt in einem RE enthaltenden Differenzierungsmedium in einem für Neuronenkulturen ausgewiesenen Spezialmedium weiter differenziert, um neuronale Zellen zu erhalten.

Bevorzugt ist dieses zur Weiterdifferenzierung verwendete Spezialmedium Neurobasal oder START V.

Erfindungsgemäß bevorzugt ist auch eine mehrstufige Kultur, bei der sich an eine Kultur in CCM enthaltendem Differenzierungsmedium eine Kultur für bis zu 2 Wochen in einem für Neuronenkulturen ausgewiesenen Spezialmedium, wie beispielsweise Neurobasal oder START V, anschließt, um retinale Zellen zu erhalten.

Erfindungsgemäß bevorzugt beträgt die Dichte der Stammzellen während der Inkubation zur Differenzierung in Retina-spezifische Zellen in Schritt b) des erfindungsgemäßen Verfahrens zwischen 0,5 × 10³ und 2,5 × 10³ Zellen pro cm², besonders bevorzugt 2 × 10³ Zellen pro cm².

Die Einstellung der Zelldichte ist entscheidend für die Differenzierung der Stammzellen in Retina-spezifische Zellen und die Veränderung der Morphologie der Stammzellen hin zu den Zielzellen. Die Gesamtzahl der zur Differenzierung einsetzbaren Stammzellen hängt von der Größe des Kulturgefäßes ab, welches die Fläche definiert, die von Zellen bewachsen werden kann. Eine Gesamtzellzahl im Bereich von 1 × 10³ bis 2,5 × 10³ Zellen ergibt sich bei Verwendung von 24 Loch-Kulturschalen mit einer zum Bewuchs bereitstehenden Fläche von 1,88 cm² pro Loch, wenn 2 × 10² bis 5 × 10³ Zellen pro Loch eingesät werden. Besonders bevorzugt wird eine Gesamtzellzahl am unteren Ende des bevorzugten Bereichs eingesetzt, da die Zellen dann bei der Aussaat vereinzelt in der Schale vorliegen und langsam proliferieren. Werden höhere Aussaatdichten von mehr als 5 × 10³ Zellen pro Loch verwendet, entstehen subkonfluente bis konfluente Zellkulturen mit stark proliferierenden Zellen, die sich jedoch nicht differenzieren, wodurch keine Veränderungen der Morphologie auftreten.

Das Chorioidea-konditionierte Medium bzw. der Retina-Extrakt enthalten entsprechend ihrer Verwendung als Zusatz zum erfindungsgemäßen Differenzierungsmedium ein oder mehrere Wachstumsfaktoren oder deren Subtypen. Der Retina-Extrakt ist zusätzlich Lieferant weiterer trophischer Faktoren der Retina und ergänzt das Differenzierungsmedium zusätzlich mit Lipoproteinen und Proteinen sowie Vitamin A und Vitamin A-Derivaten.

Das durch die Zugabe von biologischen Supplementen wie CCM, RE oder FCS in das Differenzierungsmedium verbundene potentielle Risiko einer Verunreinigung des Differenzierungsmediums mit pathogenen Keimen aus den Supplementen kann durch Ersatz dieser komplexen Zusätze vermieden werden. Der Ersatz erfolgt dabei durch Gabe definierter, in den komplexen Medien enthaltener Einzelsubstanzen, die ausgewählt sind aus der Gruppe bestehend aus Mitgliedern der FGF-Familie (FGF: "fibroblast growth factor"), Mitgliedern der NT-Familie (NT: "neurotrophin"), Mitgliedern der BMP-Familie (BMP: "bone morphogenic protein"), PDGF ("platelet-derived growth factor"), EGF ("epidermal growth factor"), BDNF ("brain-derived neurotrophic factor"), CNTF ("ciliary neurotrophic factor"), HGF ("hepatocyte growth factor") und NGF ("nerve growth factor").

Die Nennung der einzelnen Wachstumsfaktoren umfaßt erfindungsgemäß auch ihre Subtypen, deren erfindungsgemäße Verwendung ebenfalls beansprucht wird. Die Subtypen der Wachstumsfaktoren sind dem Fachmann bekannt und umfassen u.a. PDGF-AA, PDGF-BB und PDGF-AB.

In einer bevorzugten Ausführungsform der Erfindung handelt es sich bei dem Mitglied der FGF-Familie vorzugsweise um den basischen Fibroblasten-Wachstumsfaktor bFGF (FGF-2).

Die Mitglieder der Neurotrophin-Familie sind vorzugsweise NT-3 und NT-4.

Bei dem Mitglied der BMP-Familie handelt es sich vorzugsweise um BMP-4.

Die Wirkung der Wachstumsfaktoren BDNF, CNTF und Wachstumsfaktoren der NT-Familie auf das Wachstum und das Überleben von Nerven und/oder Gliazellen (BDNF) bzw. die Differenzierung verschiedener neuronaler Zelltypen (CNTF) sind sehr vielfältig.

Während NT-3 einerseits gegenüber retinalen Vorläuferzellen allgemein wie ein Mitogen wirkt und so die Entstehung eines undifferenzierten Zellpools fördert, aus dem alle retinalen Zelltypen entstehen können [DAS, et al. (2000) J Neurosci 20(8) : 2887-2895], spielt es andererseits auch bei der neuronalen Entwicklung eine Rolle und fördert, synergistisch verstärkt durch BDNF, beispielsweise das Auswachsen von Neuriten aus neuronalen Präkursorzellen [HOSSAIN, et al. (2000) Exp Neurol 175 (1) : 138-151]. Zusätzlich konnte für NT-3 eine Zellzyklus-kontrollierende Funktion in den Vorläuferzellen von sensorischen Neuronen nachgewiesen werden, deren Ausbleiben zum Zellzyklus-abhängigen Zelltod führt [ELSHAMY, et al. (1998) Neuron 21(5) : 1003-1015]. Eine Kultur von neuralen Vorläuferzellen aus dem embryonalen Striatum differenziert unter Einfluß von neurotrophen Faktoren wie NT-3 und CNTF zu bipolaren Neuronen und Oligodendrozyten, während BDNF die Differenzierung zu multipolaren Neuronen fördert [LACHYANKAR, et al. (1997) Exp Neurol 144(2): 350-360].

Neurotrophine wie NT-3, NT-4/5 bzw. BDNF besitzen eine Aktivität als "Überlebensfaktor" für Neurone des Striatums, da sie derartige Zellen bei degenerativen Erkrankungen vor dem Absterben bewahren können [PEREZ-NAVARRO, et al. (2000) J Neurochem 75(5): 2190-2199]. BDNF in Kombination mit CNTF fördert das Wachstum und die Verzweigung von Axonen nach Läsionen [LOH, et al. (2001) Exp Neurol 170(1): 72-84], während BDNF alleine die Ausdifferenzierung neuronaler Stammzellen aus dem Hippokampus zu fördern vermag [SUZUKI, et al. (2003) Biochem Biophys Res Commun 309(4): 843-847].

Die Wachstumsfaktoren der FGF- und der BMP-Familie sowie HGF wirken bei der Zellteilung von Zellen der Retina mit. Zu diesen Zellen gehören retinale Ganglionzellen (Neuronen), amakrine, bipolare und horizontale Zellen, Photorezeptorzellen (Stäbchen und Zapfen), Müller'sche Stützzellen und retinales Pigmentepithel (RPE). BMP-4 und BMP-7 als Mitglieder der BMP-Familie sind maßgeblich an der Ausbildung verschiedener Augenstrukturen wie Retina, retinalem Pigmentepithel, ciliarem Pigmentepithel und optischem Nerv beteiligt, die sich aus dem Neuroepithel ausdifferenzieren, sowie an der Schließung der Nervenverbindung zwischen Gehirn und Retina am Sehnervenkopf [LIU, et al. (2003) Dev Biol 256(1); 34-48; ADLER, et al. (2002) Development 129(13): 3161-3171]. BMP-4 übt seine steuernde Wirkung durch Förderung der Zellteilung und durch gezielte Induktion des programmierten Zelltods aus [TROUSSE, et al. (2001) J Neurosci 15;21(4) : 1292-1301] und kann sowohl verschiedene Signaltransduktionswege in Zellen aktivieren als auch die Ausdifferenzierung von Stammzellen in glatte Muskelzellen oder in Gliazellen bewirken [RAJAN, et al. (2003) J Cell Biol 161(5): 911-921]. BMPs im allgemeinen sind maßgeblich an der Ausdifferenzierung von kortikalen Stammzellen in Neurone und Astrozyten beteiligt [CHANG, et al. (2003) Mol Cell Neurosci 23(3): 414-416], während BMP-7 für die Entwicklung des Ciliarkörpers des Auges verantwortlich ist [ZHAO, et al. (2002) Development 129(19): 4435-4442]. bFGF wirkt in Abhängigkeit von der Konzentration sowohl auf Endothelzellen der Hornhaut als auch auf das retinale Pigmentepithel entweder als Mitogen oder als Differenzierungsfaktor. Ferner ist eine Wirkung des bFGF als Faktor für retinale Zellen, vor allem für Photorezeptorzellen, bekannt, der das Überleben dieser Zellen sichern kann [vgl. GU, et al. (1996) Invest Ophthalmol Vis Sci 37: 2326-2334; ITAYA, et al. (2001) Am J Ophthalmol 132: 94-100; TRAVERSO, et al. (2003) Invest Ophthalmol Vis Sci 44: 4550-4558; VALTER, et al. (2002) Growth Factors 20: 177-188; EZEONU, et al. (2000) DNA Cell Biol 19: 527-537; AKIMOTO, et al. (1999) Invest Ophthalmol Vis Sci 40: 273-279; STERNFELD, et al. (1989) Curr Eye Res 8: 1029-1037; SCHWEGLER, et al. (1997) Mol Vis 3: 10].

Der "hepatocyte growth factor" (HGF) stimuliert die Migration und Proliferation von retinalem Pigmentepithel *in vitro* und ist so förderlich bei der Wundheilung von RPE-Defekten, wobei die neu gebildeten Zellen unter Einfluß des HGF eine deutlich epitheliale Morphologie annehmen und durch Verlust von "tight junctions" frei beweglich werden [MIURA, et al. (2003) Jpn J Ophthalmol 47: 268-275; JIN, et al. (2002) Invest Ophthalmol Vis Sci 43: 2782-2790]. Ferner ist HGF ein Wachstums- und Differenzierungsfaktor für neuronale Stammzellen und fördert die Vermehrung von "Neurospheres" (aus neuralen Vorläuferzellen bestehende Zellhaufen) sowie die Ausdifferenzierung der neuralen Stammzellen in Neurone [KOKUZAWA, et al. (2003) Mol Cell Neurosci 24: 190-197].

Gegenstand der Erfindung ist ferner die Verwendung von Chorioidea-konditioniertem Medium (CCM) zur Differenzierung von Stammzellen aus dem Knochenmark in Retina-spezifische Zellen.

In einer Ausführungsform der Erfindung wird CCM zur Differenzierung von adulten mesenchymalen Stammzellen aus dem Knochenmark in Retina-spezifische Zellen verwendet.

In einer weiteren Ausführungsform der Erfindung wird CCM zur Differenzierung von adulten hämatopoetischen Stammzellen aus dem Knochenmark in Retina-spezifische Zellen verwendet.

In einer weiteren Ausführungsform der Erfindung wird CCM zur Differenzierung einer Mischung von adulten mesenchymalen und hämatopoetischen Stammzellen aus dem Knochenmark in Retina-spezifische Zellen verwendet.

Wie oben angeführt kann das Chorioidea-konditionierte Medium bei der Verwendung im erfindungsgemäßen Differenzierungsmedium ein oder mehrere Wachstumsfaktoren enthalten, ausgewählt aus der Gruppe bestehend aus Mitgliedern der FGF-Familie (FGF: "fibroblast growth factor"), Mitgliedern der NT-Familie (NT: "neurotrophin"), Mitgliedern der BMP-Familie (BMP: "bone morphogenic protein"), PDGF ("platelet-derived growth factor"), EGF ("epidermal growth factor"), BDNF ("brain-derived neurotrophic factor"), CNTF ("ciliary neurotrophic factor"), HGF ("hepatocyte growth factor"), NGF ("nerve growth factor") oder deren Subtypen.

In bevorzugten Ausführungsformen der Erfindung handelt es sich bei dem Mitglied der FGF-Familie vorzugsweise um basischen Fibroblasten-Wachstumsfaktor bFGF (FGF-2), bei den Mitgliedern der Neurotrophin-Familie vorzugsweise um NT-3 und NT-4 und bei dem Mitglied der BMP-Familie vorzugsweise um BMP-4.

Weiterer Gegenstand der Erfindung ist die Verwendung von Retina-Extrakt (RE) zur Differenzierung von Stammzellen aus dem Knochenmark in Retina-spezifische Zellen.

Erfindungsgemäß wird dabei bevorzugt RE zur Differenzierung von adulten mesenchymalen Stammzellen und/oder hämatopoetischen Stammzellen aus dem Knochenmark in Retina-spezifische Zellen verwendet.

Gegenstand der Erfindung ist auch die Verwendung von Chorioidea-konditioniertem Medium (CCM) und Retina-Extrakt (RE) zur Differenzierung von Stammzellen aus dem Knochenmark in Retina-spezifische Zellen.

**Tabelle 1: Expressionsmuster von Antigenen in verschiedenen erfindungsgemäßen Retina-spezifischen Zellen**

| Zelltyp | positives Signal | negatives Signal |
|---|---|---|
| Retinales Pigmentepithel | RPE65 | IRBP ("interphotoreceptor-retinol-binding protein") |
| | ZO-1 | CD31 |
| | Occludin | CD34 |
| | CD36 | |
| | Cytokeratin 7,8,18,19 | |
| | S-100 | |
| Photorezeptoren | Rhodopsin (Stäbchen) | |
| | Calbindin (Zapfen) | |
| | PKC (überwiegend α-Isoform, nur in Stäbchen) | PKC (Zapfen) |
| | S-Antigen (S-Ag; adulte Zellen und in späteren Entwicklungsstadien | |
| Müller-Zellen (und Astrozyten) | S-100 | PKC |
| | GFAP ("glial fibrillary acidic protein") | |
| amakrine Zellen | GABA ("gamma-aminobutyric acid") | |
| Ganglionzellen | Neurofilament | |
| bipolare Zellen | PKC | S-100 |
| horizontale Zellen | Calbindin D | |
| Choroideales Gefäßendothel | CD31 | |

Die aus Stammzellen abgeleiteten und durch das erfindungsgemäße Verfahren erhältlichen Retina-spezifischen Zellen sind ein weiterer Gegenstand der Erfindung. Als Besonderheit weisen diese erfindungsgemäßen Retina-spezifischen Zellen, die isoliert vorliegen, ein spezifisches Expressionsmuster auf (siehe Tabelle 1), das durch Expression (vgl. Spalte "positives Signal") bzw. nicht nachweisbare Expression (vgl. Spalte "negatives Signal") bestimmter, auf der Oberfläche oder intrazellulär im Cytoplasma lokalisierter Antigene gekennzeichnet ist. Bis auf die Antigene RPE65 und Rhodopsin sind die untersuchten Antigene nicht spezifisch für den Zelltyp. Da die untersuchten Antigene jedoch gewebespezifisch für retinales und auch neurales Gewebe sind, ermöglichen sie zusätzlich zu den morphologischen Unterschieden bei den Zellen eine Differenzierung der Retina-spezifischen Zellen von den Stammzellen, aus denen sie hervorgegangen sind (siehe oben) durch Immunfärbung und Interpretation der charakteristischen Färbeergebnisse (Positiv-/Negativfärbung).

Durch die erfindungsgemäßen Retina-spezifischen Zellen wird ein weites Feld für die genetische Modifikation und Therapie eröffnet. Gemäß einer Ausführungsform der Erfindung sind die isolierten Stammzellen aus dem Knochenmark per se oder die letztlich daraus differenzierten Retina-spezifischen Zellen mit einem oder mehreren Genen transfiziert oder transduziert. In einer bevorzugten Ausführungsform der Erfindung werden die isolierten Stammzellen im Anschluß an die Isolierung aus dem Knochenmark bzw. im Zuge des Differenzierungsverfahrens im Anschluß an die Expansion im Verfahrensschritt a), im Anschluß an die Differenzierung im Verfahrensschritt b) oder die daraus differenzierten Retina-spezifischen Zellen mit einem oder mehreren humanen, Retina-spezifischen Genen als Transgene transfiziert.

Unter den Retina-spezifischen Transgenen werden hierin solche Gene verstanden, die natürlicherweise in gesunden Retinazellen, aber nicht in den undifferenzierten oder differenzierten Stammzellen exprimiert werden.

Autologe Stammzellen und die Netzhautzellen eines Patienten weisen identische Defekte in ihren Genomen auf, die bei Aktivierung der betroffenen Gene durch ausbleibende oder fehlerhafte Expression zur Etablierung eines Krankheitsbildes führen, in Falle von Netzhautzellen z.B. in der Netzhaut. Eine gezielte Gentherapie derartiger Krankheiten, z.B. der Retinitis pigmentosa, kann durch Transfektion der erfindungsgemäß verwendeten Stammzellen oder der daraus differenzierten Retina-spezifischen Zellen vor einer Transplantation der Zellen mit einer gesunden Kopie des defekten Gens erfolgen. Werden im Verlauf des erfindungsgemäßen Verfahrens Gene in die Stammzellen eingeführt, bleiben diese bevorzugt auch in den sich aus den Stammzellen differenzierenden Retina-spezifischen Zellen erhalten und exprimieren das transfizierte Gen nach Transplantation in den Empfänger auch am Ort der Transplantation. Verfahren zur Transfektion von Zellen mit Transgenen sind dem Fachmann wohlbekannt [vgl. SAMBROOK, J. et al. (2001) Molecular Cloning: A Laboratory Manual. Cold Spring Harbor Laboratory Press].

Die Genkonstrukte, die zur Transfizierung der Stammzellen oder der daraus differenzierten Retina-spezifischen Zellen verwendet werden, können auf verschiedene, dem Fachmann bekannte Arten gestaltet und zusammengesetzt sein.

Idealerweise sollten defekte oder fehlende Gene in ihrem natürlichen Kontext repariert bzw. ersetzt werden, was nach dem heutigen Stand der Technik jedoch praktisch nicht realisierbar ist. Daher müssen die fehlenden bzw. defekten Gene zusätzlich in das Genom der Stamm- bzw. Retina-spezifischen Zellen eingebracht und dort ektop exprimiert werden. Um eine stabile Expression sowie die Weitergabe der eingebrachten Gene an die Tochterzellen im Zuge der Zellteilung zu gewährleisten, sollten nach dem Stand der Technik retrovirale [Baum et al., Curr Opin Mol Ther. 1999 Oct; 1(5):605-612] oder lentivirale [Trono, Gene Ther 2000; 7: 20-33], evtl. auch AAV-Vektoren [Monahan & Samulski, Gene Ther 2000; 7:24-30] als Genfähren benutzt werden. Die Viren, von denen diese Vektoren abgeleitet sind, zeichnen sich dadurch aus, dass sie natürlicherweise stabil in das Zielzellgenom integrieren und mithin wie eigene Gene weitervererbt werden.

Nach einer Transduktion mit herkömmlichen von γ-Retroviren abgeleiteten Vektoren würde eine ungesteuerte Expression des eingebrachten Transgens erfolgen. Die Höhe dieser Expression kann jedoch vorab durch die Wahl geeigneter viraler Promotoren in relativ großen Grenzen bestimmt werden [Baum et al., Curr Opin Mol Ther. 1999 Oct; 1(5):605-612; Wahlers et al., Gene Ther. 2001 Mar; 8(6):477-486]. Die Nutzung sog. SIN ("selfinactivating") Vektoren erlaubt es dagegen, die viralen Promotoren durch jeden anderen Promotor der Wahl zu ersetzen [Kraunus et al., Gene Ther. 2004 Nov; 11(21):1568-1578]. Aus Gründen der Biosicherheit werden bei lentiviralen (in der Regel HIVabgeleiteten) Vektoren ausschließlich SIN-Konstrukte benutzt. Da SIN-Vektoren die viralen Promotor- und Enhancerelemente fehlen, könnten sie möglicherweise mit einem geringeren Risiko schädlicher Nebenwirkungen ("Insertionsmutagenese") behaftet sein [von Kalle et al., Stem Cell Clonality and Genotoxicity in Hematopoietic Cells: Gene Activation Side Effects Should Be Avoidable. Seminars in Hematology, in press]. Für den hier gegebenen Kontext sind SIN-Vektoren vor allem dadurch interessant, dass sie den Einsatz genspezifischer [Moreau-Gaudry et al., Blood. 2001; 98:2664-2672] oder aber regulierbarer bzw. induzierbarer Promotoren erlauben. Der Einsatz Retina-spezifischer Promotoren wäre sicher die optimale Lösung. Induzierbare Systeme beruhen zur Zeit zumeist auf dem Tetrazyklin-System von Gossen & Bujard [Proc Natl Acad Sci USA. 1992; 89(12):5547-51]. Mit derartigen Systemen kann während der Proliferation der Stammzellen bzw. der Differenzierung der Stammzellen in Retina-spezifische Zellen durch Zugabe des jeweiligen inhibierenden Stoffes in das jeweilige Kulturmedium die Expression des Transgens während der Kultur unterbunden werden. Wird dem Patienten nach Transplantation der Retina-spezifischen Zellen dieser Stoff nicht zugeführt, wird die Inhibierung beendet, der Promoter aktiviert und das Transgen exprimiert.

Durch die Transfektion mit einem oder mehreren Fremdgenen können einerseits Gene in die Zellen eingeführt werden, die für die Aufrechterhaltung zelltypischer metabolischer Leistungen in den Retina-spezifischen Zellen erforderlich sind, andererseits ist aber auch eine Transfektion von Genen eingeschlossen, die den Retina-spezifischen Zellen neue Funktionen verleihen oder die Zelle markieren. In einer besonders bevorzugten Ausführungsform der Erfindung werden die Zellen mit dem "green fluorescent protein" (GFP), dem "enhanced green fluorescent protein" (eGFP) oder dem Gen lacZ als Marker- oder Reportergen zur Markierung der Zellen transfiziert [vgl. ALLAY, J.A. et al. (1997) Hum Gene Ther 8: 1417; AYUK, F. et al. (1999) Gene Ther 6: 1788-1792; FEHSE, B. et al. (1998) Gene Ther 5: 429-430].

Die Erfindung betrifft weiterhin Zellzubereitungen, die erfindungsgemäße Retina-spezifische Zellen als isolierte Zellen enthalten. Solche Zellzubereitungen sind für die Lagerung oder den Transport der Zellen einsetzbar.

Zellzubereitungen können isolierte vitale Retina-spezifische Zellen gemäß der Erfindung, die durch fehlende oder nicht nachweisbare Expression von Markern ausgewählt aus der Gruppe bestehend aus IRBP und CD34 bzw. durch die Expression von mindestens einem der Marker ausgewählt aus der Gruppe bestehend aus RPE65, ZO-1, Occludin, CD36, Cytokeratin 7, Cytokeratin 8, Cytokeratin 18, Cytokeratin 19, S-100, Rhopopsin (in Stäbchen), Calbindin (in Zapfen), PKC, S-Antigen, GFAP, GABA und Neurofilament, gekennzeichnet sind, in einer Menge von mindestens 1, bevorzugt von 1 - 50 %, in besonders bevorzugter Weise von 50 bis 70%, und in äußerst bevorzugter Weise von 70 bis 90%, bezogen auf die Gesamtzahl der in dem Präparat vorhandenen Zellen in einem geeigneten Medium, enthalten, wobei in dem vorgenannten Wertebereich alle ganzzahligen Werte (d.h. 11, 12, 13, ... 90 %) ausdrücklich eingeschlossen sind. Bevorzugt sind Zellsuspensionen in einem zellverträglichen Zellkultur- oder Transportmedium, wie z.B. einem Standardmedium ausgewählt aus der Gruppe bestehend aus RPMI, Medium 199, DMEM (low glucose; dieses Medium entspricht dem Modified Eagles's Medium (Gibco 31885)) mit oder ohne HEPES als Zusatz und Iscove's Medium jeweils allein oder als 1/1-Mischung mit Ham's F12 Nutrient Mixture. Das Medium kann ferner ein Spezialmedium sein ausgewählt aus der Gruppe bestehend aus Medium Human Endothelial-SFM (Gibco 11111), START V (Biochrom F8075) und Neurobasal- bzw. Neurobasal-A Medium (Gibco 21103 bzw. 10888) mit oder ohne Ham's F12 Nutrient Mixture als Zusatz.

In Frage kommen auch tiefgefrorene Zellzubereitungen, bei denen die Zellen durch Zentrifugation sedimentiert und beispielsweise in 90 % FCS und 10 % DMSO aufgenommen wurden. Dem Kryomedium werden 10% Methylcellulose oder DMSO als Hilfsstoff zugegeben, um das Überleben der Zellen während der Kryokonservierung zu unterstützen. Bei serumfreier Behandlung der Zellen müssen zusätzlich schützende Proteine zugegeben werden, an die empfindliche Proteine anhaften können und dadurch während der Kryokonservierung geschützt sind. Bevorzugt werden diese als Albumin zugegeben. Statt in Differenzierungsmedium können die Zellen auch in serumfreiem Kryomedium (z.B. Cryo-SFM (Promocell C-29910) aufgenommen werden. Dabei sind Kryomedien solche Medien, die ein Tieffrieren der Zellen ohne Beschädigung der Zellen erlauben.

In einer Ausführungsform der Erfindung werden nach der Differenzierung die differenzierten Retina-spezifischen Zellen von undifferenzierten Stammzellen abgetrennt, um eine größtmögliche Anreicherung erfindungsgemäßer Retina-spezifischer Zellen bei gleichzeitiger Abreicherung der nicht-differenzierten Stammzellen zu erzielen. Die Trennung der undifferenzierten Stammzellen von den differenzierten Retina-spezifischen Zellen erfolgt mit Hilfe von (Oberflächen)antigenen, die spezifisch auf den (an)differenzierten Retina-spezifischen Zellen, aber nicht oder nicht nachweisbar auf den undifferenzierten Stammzellen exprimiert werden. Antigene, die für eine derartige Trennung verwendet werden können, sind beispielsweise CD36 oder S-100 sowie alle Antigene aus der Spalte "positives Signal" (siehe Tabelle 1). Durch die Trennung der Zellen wird weitestgehend verhindert, daß sich in der Menge der Zellen neben den Retina-spezifischen Zellen mit rein proliferativer Kapazität quantitativ große Mengen differenzierungsfähiger Zellen befinden. Ferner kann so gewährleistet werden, daß eingestellte Zellzahlen, beispielsweise bei der Herstellung einer pharmazeutischen Zusammensetzung, tatsächlich die erfindungsgemäßen Retina-spezifischen Zellen repräsentieren.

In einer weiteren bevorzugten Ausführungsform der Erfindung erfolgt nach der Differenzierung keine Trennung der differenzierten Retina-spezifischen Zellen von undifferenzierten Stammzellen, sondern eine Anreicherung der differenzierten Zellen bzw. eine Abreicherung der undifferenzierten Stammzellen. Eine derartige An- bzw. Abreicherung erfolgt ebenfalls mit Hilfe von spezifischen Oberflächenantigenen.

Beispiele für im Stand der Technik bekannte Verfahren mittels derer anhand bestimmter Oberflächenmarker Zellen sortiert werden können, umfassen das "Immuno magnetic bead sorting" [vgl. ROMANI, et al. (1996) J Immunol Methods 196: 137-151], das "Fluorescence-Activated Cell Sorting" (FACS) und das "Magnetic-Activated Cell Sorting" (MACS) [loc. cit.]. Weitere derartige Verfahren sind dem Fachmann bekannt.

In einer Ausführungsform der Erfindung werden die erfindungsgemäßen Retina-spezifischen Zellen per se zur Herstellung einer pharmazeutischen Zusammensetzung zur Behandlung von Krankheiten, die mit erworbener oder angeborener Dysfunktion der Zellen des retinalen Pigmentepithels, der Zellen der benachbarten Strukturen der gesamten Retina und der Chorioidea sowie weiterer Augengewebe assoziiert sind, oder zur Regeneration des Sehnerven (Nervus opticus) z.B. bei oder nach Glaukomschaden eingesetzt.

Das Knochenmark, aus dem die Stammzellen isoliert werden, kann autologen oder allogenen Ursprungs sein. Der Begriff "autolog" bezeichnet Gewebe oder Zellen, die dem gleichen Individuum entnommen wurden, das die differenzierten Retina-spezifischen Zellen als Transplantat erhalten soll. Ein allogener Ursprung deutet an, daß der Spender des Knochenmarks und der Empfänger der Retina-spezifischen Zellen, die aus dem Knochenmark differenziert wurden, unterschiedlich sind, jedoch zur gleichen Spezies gehören, z.B. sind Spender und Empfänger Menschen.

Gemäß einer besonders bevorzugten Ausführungsform der Erfindung sind die Retina-spezifischen Zellen autologe Zellen, d.h. die Stammzellen aus dem Knochenmark stammen von dem Patienten, der mit den Retina-spezifischen Zellen, die aus diesen Stammzellen differenziert wurden, behandelt werden soll. In einem solchen Fall verursacht die Gabe von aus Stammzellen differenzierten Retina-spezifischen Zellen keine immunologischen Problemen in Form einer Zellabstoßung, da die Gewebemerkmale der Zellen und des Empfängers identisch sind.

Die pharmazeutischen Präparate können die erfindungsgemäßen Retina-spezifischen Zellen, d.h. an- und/oder ausdifferenzierte Zellen, in einem physiologisch verträglichen Medium suspendiert enthalten. Geeignete Medien sind beispielsweise Standardmedien ausgewählt aus der Gruppe bestehend aus RPMI, Medium 199, DMEM (low glucose; dieses Medium entspricht dem Modified Eagles's Medium (Gibco 31885)) mit oder ohne HEPES als Zusatz und Iscove's Medium jeweils allein oder als 1:1-Mischung mit Ham's F12 Nutrient Mixture, oder Spezialmedien ausgewählt aus der Gruppe bestehend aus Medium Human Endothelial-SFM, START V und Neurobasal- bzw. Neurobasal-A Medium mit oder ohne Ham's F12 Nutrient. Bei Verwendung der Spezialmedien zur Herstellung einer pharmazeutischen Zusammensetzung muß darauf geachtet werden, daß die Medien für diese Verwendung geeignet sind und keine Hormone, Peptide oder dergleichen enthalten, auf die der Patient empfindlich reagieren könnte. Unbedingt zu beachten ist, daß das zur Transplantation benutzte Medium kein Serum enthalten darf. Ersatzweise können physiologische Lösungen, z. B. Ringerlösung, eingesetzt werden.

Die Retina-spezifischen Zellen gemäß der Erfindung, die durch mindestens einen der Marker ausgewählt aus der Gruppe bestehend aus RPE65, ZO-1, Occludin, CD36, Cytokeratin 7, Cytokeratin 8, Cytokeratin 18, Cytokeratin 19, S-100, Rhopopsin (in Stäbchen), Calbindin (in Zapfen), PKC, S-Antigen, GFAP, GABA und Neurofilament gekennzeichnet sind, liegen in derartigen pharmazeutischen Zusammensetzungen bevorzugt in einer Menge von mindestens 50%, bevorzugt mindestens 60% bezogen auf die Gesamtzahl der in dem Präparat vorhandenen Zellen vor, wobei in dem vorgenannten Wertebereich alle ganzzahligen Werte (d.h. 51, 52 ... 59 und 61, 62 ... 99, 100) ausdrücklich eingeschlossen sind. Die pharmazeutischen Präparate können gegebenenfalls weitere pharmazeutisch verträgliche Hilfs- und/oder Trägerstoffe enthalten.

In einer weiteren bevorzugten Ausführungsform der Erfindung liegen in den pharmazeutischen Präparaten mindestens 1 × 10⁴ erfindungsgemäße Retina-spezifische Zellen pro µl vor. Bevorzugt liegen jedoch nicht mehr als 5 × 10⁴ erfindungsgemäße Retina-spezifische Zellen pro µl vor, um ein Verklumpen der Zellen zu vermeiden.

Bevorzugte Applikationsformen für die in vitro differenzierten Retina-spezifischen Zellen sind Injektion, Infusion oder Implantation der Zellen in einen spezifischen Zellverband des Auges, um zu erreichen, daß die Zellen dort einerseits durch den direkten Kontakt mit dem Zellverband anwachsen und durch entsprechende gewebegemäße Differenzierung Funktionen des geschädigten Gewebes übernehmen.

Eine besonders bevorzugte Applikationsform ist die Injektion der *in vitro* differenzierten Retina-spezifischen Zellen. Diese Implantation erfolgt bevorzugt lokal intraokular.

Besonders bevorzugt erfolgt die lokal intraokulare Applikation in die Retina (intraretinal, vgl. GUO, Y. et al. (2003) Invest Ophthalmol Vis Sci 44(7) : 3194-3201), unter die Retina (subretinal, vgl. WOJCIECHOWSKI, A.B. et al. (2002) Exp Eye Res 75(1): 23-37) oder nahe an der Retina in den Glaskörper (intravitreal, vgl. JORDAN, J.F. et al. (2002) Graefe's Arch Clin Exp Ophthalmol 240(5): 403-407).

Eine weitere bevorzugte Ausführungsform der Erfindung betrifft die systemische Infusion der erfindungsgemäßen *in vitro* differenzierten Retina-spezifischen Zellen über die Blutbahn zur Anreicherung der Zellen in der Retina.

Bevorzugte Beispiele für in diesem Zusammenhang relevante Indikationen sind Retinitis pigmentosa, altersabhängige Makuladegeneration oder Glaukom. Der Begriff "Glaukom" bezeichnet dabei eine Reihe von Degenerationen der Nervenfasern und des Sehnerven, die einem zumeist anomalen intraokularen Druck zugeschrieben werden. Gekennzeichnet ist dies durch einen Untergang der Ganglienzellen der Retina und der Nervenfasern sowie eine Atrophierung des Sehnerven. Mit dem Begriff "Glaukom" werden erfindungsgemäß alle Arten von Glaukomen erfaßt, d.h. Hoch-, Normal-Niederdruckglaukom, Offenwinkelglaukom, PEX-Glaukom etc.

Die erfindungsgemäße Behandlung des Glaukoms umfaßt den Ersatz zerstörter Ganglien- und Nervenzellen der Retina und im Sehnerven durch Gabe erfindungsgemäß in Retina-spezifische Zellen anoder ausdifferenzierte Stammzellen aus dem Knochenmark zur Bildung eines Ersatzes der zerstörten Zellen.

Mit den erfindungsgemäß aus hämatopoetischen Stammzellen differenzierten Retina-spezifischen Zellen sind auch die mit einer Diabeteserkrankung einhergehenden Schäden an der Chorioidea (Retinopathia diabetica) behandelbar. Eine Gabe der erfindungsgemäßen Zellen stabilisiert die infolge der Diabetes brüchig gewordenen Gefäße und verringert oder verhindert dadurch das Auftreten von Netzhautblutungen.

Folglich sind bevorzugte Ausführungsformen der Erfindung die Verwendung der Retina-spezifischen Zellen zur Herstellung pharmazeutischer Zusammensetzungen zur Behandlung von Retinitis pigmentosa, altersabhängiger Makuladegeneration oder Glaukom.

Weiterhin erfindungsgemäß bevorzugt ist die Verwendung der aus hämatopoetischen Stammzellen an- oder ausdifferenzierten Retina-spezifischen Zellen zur Herstellung einer pharmazeutischen Zusammensetzung zur Behandlung von Erkrankungen, die durch eine Degeneration der Gefäßstrukturen der Chorioidea gekennzeichnet sind, z.B. der Retinopathia diabetica bei Diabetes.

Die Zellen können, wie oben beschrieben, autologen oder allogenen Ursprungs sein, d.h. das Knochenmark, aus dem die mesenchymalen oder hämatopoetischen Stammzellen isoliert wurden, stammt aus dem Körper des Empfängers oder eines Vertreters seiner Art.

Die Erfindung wird nachfolgend anhand von Beispielen erläutert und beschrieben, ohne auf diese Ausführungsbeispiele beschränkt zu sein:

### Beispiel 1

### Isolierung von Knochenmarkzellen

Adulte mesenchymale Stammzellen wurden aus Knochenmarkproben (Aspiraten) gewonnen, die einem lebenden Spender im Verlauf eines kleinen Eingriffs entnommen wurden. Die Stammzellen wurden aus der Probe durch Zentrifugation über einen Ficollgradienten (Biochrom KG, "Biocoll Separation Solution, isotonic solution; Dichte 1,077 g/ml) abgetrennt. Die Zellen aus der Schicht der mononukleären Zellen wurden in einem Kulturmedium (DMEM, low glucose) ergänzt mit 10% fötalem Kälberserum (FCS) resuspendiert und in unbeschichteten "tissue culture-treated plastic"-Kulturschalen (Polystyrol) kultiviert.

Die erste Kultur nach der Isolierung der Zellen erfolgt im allgemeinen in 24-Lochplatten. Je nach Größe der Kultur kommen auch 12-Lochplatten, 6-Lochplatten, T25-Kulturflaschen oder T75-Kulturflaschen in Betracht.

Die durch dieses Verfahren erhaltenen Zellkulturen können in DMEM (low glucose)-Medium mit 10% FCS für mehrere Monate kultiviert werden, indem sie alle 7 bis 14 Tage in Abhängigkeit von der Aussaatdichte, dem Spendereinfluß, dem Alter der Kultur und bei Erreichen der Subkonfluenz (60-80 %) passagiert werden (siehe Beispiel 2).

### Beispiel 2

### Passagierung der mesenchymalen Stammzellen

Zur Passagierung der mesenchymalen Stammzellen wurden das Kulturmedium und nicht-anhaftende Zellen von den plastikadhärent wachsenden mesenchymalen Stammzellen durch Absaugen oder Abnahme entfernt. Die an der Kulturschale haftenden adhärent wachsenden mesenchymalen Stammzellen wurden 1-2 mal mit PBS (das keine Calcium- oder Magnesiumionen enthielt!) gespült, um weitere nicht-adhärente Zellen zu entfernen. Es schloß sich eine 1-minütige Inkubation bei Raumtemperatur in einer Trypsin/EDTA-Lösung an (Trypsin 0,02 %, EDTA 0,05 % in Calcium- oder Magnesiumionen-freiem PBS). Nach Abschluß der Inkubation wurde die Trypsin/EDTA-Lösung wieder abgesaugt und die Zellen weitere 2-3 Minuten bei Raumtemperatur belassen. Anschließend wurde das Kulturgefäß vorsichtig durch Klopfen mit der Hand erschüttert, um durch die mechanische Beanspruchung die Zellen von der Oberfläche des Kulturgefäßes zu lösen. Die losgelösten Zellen wurden in DMEM low glucose/10% FCS suspendiert.

Zur Bestimmung der Zellzahl wurden entweder 10 µl der Suspension mit 10 µl einer Trypanblaulösung vermischt, in eine Neubauerkammer pipettiert und unter dem Mikroskop avitale (gefärbte Zellen) und vitale (nichtgefärbte) Zellen gezählt, oder es wurden 0,5 ml der Suspension mit 12,5 bis 19,5 ml einer isotonen Salzlösung (speziell zur Anwendung in einem "Coulter Counter"-Zellzählgerät) verdünnt und diese in einem "Coulter Counter"-Zellzählgerät gezählt.

Die Gesamtzahl vitaler Zellen wird in beiden Fällen unter Einbeziehung der Verdünnung rechnerisch ermittelt. Zur Passagierung wurden die Zellen nun in entsprechende unbeschichtete Kulturgefäße ausgesät und im gleichen Kulturmedium weiter kultiviert, das zur Aussaat verwendet wurde. Eventuell ist dazu eine weitere Verdünnung der Zellsuspension mit Kulturmedium notwendig.

Die Zellsuspension kann ferner durch Zentrifugation sedimentiert, die sedimentierten Zellen in Einfriermedium (90 % FCS + 10 % DMSO) suspendiert und in flüssigem Stickstoff kryokonserviert werden.

### Beispiel 3

### Herstellung von Retina-spezifischen Zellen durch Einsatz von CCM

Für die Differenzierung adhärent wachsender mesenchymaler Stammzellen nach Isolierung aus dem Knochenmark (siehe Beispiel 1 und 2), wurden zuerst 4 bis 8 ml Chorioidea-konditioniertes Medium (CCM) generiert. Zur Herstellung von 4 ml CCM wurden 2 Augen (entspricht einem Augenpaar) eines allogenen Donors wie folgt behandelt:

Zuerst wurden das anteriore Segment, der Glaskörper und die neurosensorische Netzhaut des Auges entfernt, gefolgt von einer Präparation der Chorioideae und/oder deren Fragmenten mit einer Schere und einer Pinzette [vgl. VALTINK, M. et al. (1999) Graefe's Arch Clin Exp Ophthalmol 237: 1001-1006; VALTINK, M. & ENGELMANN, K. (2002) In: WILHELM, F., DUNCKER, G.I.W., BREDEHORN, T. (Hrsg.) Augenbanken. Walter de Gruyter Verlag Berlin New York, S. 75-87]. In der Regel ist die Chorioidea noch vollständig. Durch Waschen mit 2 ml phosphatgepufferter Salzlösung (PBS) pro Chorioidea wurden Blut, lose anhaftende, avitale Zellen und Gewebefragmente entfernt, die bei der weiteren Behandlung der Chorioidea stören würden. Es schloß sich eine Inkubation in einer Kollagenaselösung (1:1 Kollagenase IA und IV [vgl. VALTINK, M. et al. (1999) Graefe's Arch Clin Exp Ophthalmol 237: 1001-1006; VALTINK, M. & ENGELMANN, K. (2002) In: WILHELM, F., DUNCKER, G.I.W., BREDEHORN, T. (Hrsg.) Augenbanken. Walter de Gruyter Verlag Berlin New York, S. 75-87]; Endkonzentration 0.5 mg/ml; 2 ml Lösung pro Chorioidea) in einem Brutschrank unter 5% CO₂ bei 37°C für etwa 4 bis 16 Stunden an, um Zellen des retinalen Pigmentepithels von dem chorioidealen Gewebe abzulösen. Bei Verwendung höherer Kollagenase-Endkonzentrationen (z.B. 1 mg/ml) genügt eine Inkubationszeit von 1 bis 4 Stunden. In einigen Fällen ist die Chorioidea durch die Enzymaktivität der Kollagenase aufgelockert und zerfällt bei Überführung in neues Medium.

Der nachfolgende Konditionierungsprozeß wird dadurch nicht beeinträchtigt. Die Enzymaktivität wurde nachfolgend durch Zugabe eines Überschusses von Serum-enthaltendem Kulturmedium (DMEM + FCS, siehe Beispiel 1) gestoppt. Danach wurde das chorioidale Gewebe in 2 ml Kulturmedium bestehend aus F99-Medium ergänzt mit 1 % FCS pro Chorioidea, also 4 ml Medium pro Augenpaar, überführt. Das Gewebe wurde in einem Brutschrank unter 5% CO₂ bei 37°C für 4 Tage inkubiert.

Die Enzymaktivität der Kollagenase wird durch Zugabe eines Überschusses von Serum-enthaltendem Kulturmedium nur partiell gestoppt, da kommerzielle Kollagenasen neben der proteolytischen Spaltung von Kollagenen als Hauptaktivität weitere, schwer inaktivierbare proteolytische Aktivitäten aufweisen, die sich gegen andere Proteinstrukturen richten. Diese nicht inaktivierbare Restaktivität ist jedoch gering und hat keinen Einfluß auf die Bildung des konditionierten Mediums und dessen Verwendung zur Zellkultivierung und Differenzierung.

Während dieser Inkubation bildete sich CCM als Überstand. Dieser wurde von dem chorioidalen Gewebe durch Zentrifugation bei Raumtemperatur mit 300 × g für 10 Min abgetrennt. Der erhaltene Überstand wurde direkt als Zusatz zur Differenzierung verwendet oder bei - 20°C tiefgefroren.

Ca. 15.000 Stammzellen, die aus Knochenmark stammen (siehe Beispiel 1 und Beispiel 2 für mesenchymale Stammzellen) und maximal bis Passage 6 kultiviert wurden (siehe Beispiel 2), wurden mit 5 ml Medium F99 (dies ist eine 1:1-Mischung aus Medium 199 und Ham's F12 Nutrient Mixture), welches mit 1 bis 10 % FCS, 1 µg/ml Insulin, 1 mmol/l Natriumpyruvat und 10 % CCM supplementiert ist, in einer T25-Kulturflasche für 14 bis 21 Tage inkubiert. 2 bis 3 mal pro Woche wurde das Differenzierungsmedium gewechselt, wodurch sich eine Gesamtmenge von ca. 30 bis 45 ml Differenzierungsmedium ergibt, die zur Differenzierung einer Spenderkultur benötigt wird.

Nach ungefähr 5 Tagen wurde bei den Zellen eine Abnahme der Teilungsgeschwindigkeit und deutliche morphologische Veränderungen hin zu einer stellaren Morphologie beobachtet (vgl. Figur 2). Zusätzlich war eine Ansammlung von dunklen Granula um den Kern zu beobachten.

Nach 10 bis 14 Tagen in Kultur begannen die Zellen eine neuronale Morphologie auszubilden, mit dendritischen, vielfach verzweigten Ausläufern, häufig begleitet von einer Bildung von Podien an Kontaktstellen mit benachbarten Zellen (vgl. Figur 3b und Figur 4).

Nach 19 Tagen in Kultur wurde zur weiteren Charakterisierung der Zellen nach Entfernen des Kulturmediums zu nicht fixierten bzw. zu vorab bei 4°C mit 5 %igem Formalin fixierten Zellen eine Lösung des Aminosäurederivates L-3,4-Dihydroxyphenylalanin (L-DOPA, 0,1 %-ige Lösung in PBS mit neutralem pH, entspricht 1 mg/l) für den Nachweis aktiver Tyrosinase, dem Schlüsselenzym der Melanogenese, gegeben und für 45 min bei 37°C inkubiert. Nach Beendigung der 45-minütigen Inkubation wurde die Lösung jeweils erneuert, bis eine Gesamtdauer der Inkubation von 3 Stunden erreicht war, wobei alle 30 min auf das Fortschreiten der Reaktion geachtet wurde.

Mit Hilfe dieses Nachweises wird überprüft, ob die Stammzellen auch in pigmentierte Zelltypen, wie beispielsweise Zellen des retinalen Pigmentepithels oder Melanozyten, zu differenzieren vermögen. Die Fähigkeit der Zellen zur Pigmentierung über den Tyrosinaseweg ist dabei das entscheidende Differenzierungskriterium. Der Nachweis ist positiv, wenn aus Melanin bestehende schwarzbraune Körnchen als Ablagerungen sichtbar werden, die aus dem zugegebenen L-DOPA durch das in den Zellen vorhandene Enzym Tyrosinase und seine Abkömmlinge sowie der nachfolgenden Enzyme in dieser Reaktionskette gebildet werden.

Derartige Zellen waren nach 19 Tagen in Kultur nachweisbar.

### Beispiel 4

### Herstellung von Retina-spezifischen Zellen durch Einsatz von RE

Zur Differenzierung der aus Knochenmark isolierten adhärent wachsenden mesenchymalen Stammzellen (siehe Beispiele 1 und 2) bzw. nicht-adhärent wachsenden hämatopoetischen Stammzellen (siehe Beispiele 1 und XY), wurde Retina-Extrakt (RE) verwendet, der durch Homogenisieren von Retinas erzeugt wurde.

RE wurde wie folgt hergestellt:

Aus 10 humanen Spenderaugen wurde die neurosensorische Retina präpariert. Dazu wurde zuerst das anteriore Segment und dann der Glaskörper der Augen entfernt. Die neurosensorische Netzhaut der Augen wurde anschließend mit einer Pinzette angehoben und mit einer Schere am Sehnervenkopf abgetrennt. Die erhaltenen Retinas wurden als Ganzes in einem Gefäß mit PBS auf ein Volumen von 50 ml aufgefüllt und unter Zusatz von Proteinasehemmern (z.B. 1 Tablette Complete Protease Inhibitor Cocktail (Roche) auf 50 ml Homogenat) in einem Hand- oder Gewebe-Homogenisator aus Glas auf Eis homogenisiert. Der Überstand, der den RE repräsentiert, wurde durch eine Zentrifugation mit 500 × g für 15 min und eine weitere Zentrifugation mit 10000 × g für 45 min gewonnen. Der RE wurde anschließend durch einen 0,22 µm-Sterilfilter sterilfiltriert.

Die Differenzierung der passagierten Stammzellen mit RE enthaltendem Differenzierungsmedium erfolgte analog zu Beispiel 3. Dem Differenzierungsmedium wurde jedoch abweichend 1 % RE statt des CCM zugegeben. Die Differenzierung der Stammzellen in Retina-spezifische Zellen erfolgte während einer Kultur über 2 bis 3 Wochen im Differenzierungsmedium.

### Beispiel 5

### Analyse der Zusammensetzung des Chorioidea-konditionierten Mediums über MALDI-TOF

Obwohl die Wirkung des CCM auf die Proliferation und Differenzierung der mesenchymalen Stammzellen (MSC) und der Zellen des retinalen Pigmentepithels (RPE) gezeigt werden konnte (siehe Beispiele 3 und 4), war die genaue Zusammensetzung dieses Mediums zunächst nicht bekannt.

Um die Zusammensetzung des CCM zu ermitteln, wurde der Überstand aus der Chorioidea-Kultur nach Beispiel 3 mittels Gelfiltration über eine Superdex^{®}-Säule (Pharmacia Biotech) in 80 Fraktionen aufgetrennt. Durch Bestimmung des Proteingehalts der einzelnen Fraktionen in einem Chromatographen mittels Messung der Absorption bei 214 und 280 nm wurde ein Chromatogramm der einzelnen Fraktionen erstellt (siehe Figur 5).

Anhand der in dem Chromatogramm sichtbaren Signalpeaks wurden die Fraktionen vereint, die jeweils einer Gruppe von Peptiden/Proteinen zugeordnet werden konnten. Beispielsweise wurden jeweils die Fraktionen 22 - 37, die Fraktionen 38 - 41 und die Fraktionen 42 - 46 getrennt vereinigt. Die Fraktionen 22 - 37 enthalten kleinere Peptid-/Proteinmoleküle, die vor der Konditionierung des Mediums in dieser Konzentration nicht vorhanden waren und neu synthetisierte kleinere Peptide/Proteine oder Abbauprodukte des Serums sind. Die Fraktionen 38 - 41 enthalten Peptide/Proteine aus dem größten Peak, die schon im Medium vor der Konditionierung vorhanden war, deren Menge durch die Inkubation mit den Chorioidea jedoch erhöht wurde.

Dies deutet darauf hin, daß durch die Konditionierung nicht alle Serumproteine verbraucht worden sind, die dem Medium ursprünglich zugefügt wurden. Die Fraktionen 42 - 46 enthalten Peptide/Proteine, die im Medium vor der Konditionierung nicht vorhanden waren. Die Größe der in diesem Peak enthaltenen Peptide/Proteine legt nahe, daß sie keine Abbauprodukte des Serums darstellen, sondern aus der Chorioidea stammen müssen und bei der Konditionierung des Mediums in dieses abgegeben wurden.

Die Fraktionen wurden nach Vereinigung auf ihre biologische Aktivität getestet, indem sie einem Kulturmedium zugesetzt wurden, das zur Züchtung von humanen mesenchymalen Stammzellen und humanen retinalen Pigmentepithelzellen verwendet wurde.

Dazu wurden normale humane RPE-Zellen zweier Spender aus der ersten und dritten Passage mit einer Aussaatdichte von 500 Zellen pro Vertiefung in 12 Loch-Kulturschalen mit F99-Medium, das mit 10% FCS supplementiert wurde, ausgesät und über Nacht inkubiert, damit die Zellen an der Schale adhärieren konnten. Anschließend wurde das Medium gegen die Testmedien ausgetauscht, die sich aus F99, 5% FCS und den Fraktionen aus der Fraktionierung des CCM zusammensetzen. Als Negativkontrolle wurde F99 versetzt mit 5% FCS ohne Zusatz einer CCM-Fraktion, als Positivkontrolle F99 versetzt mit 5%. FCS und CCM eingesetzt. Als Positivkontrolle wurde CCM einerseits ohne Fraktionierung als Ganzes, andererseits auch nach Fraktionierung und erneuter Vereinigung eingesetzt. Es wurden jeweils 3 Vertiefungen mit dem selben Medium versehen, so daß jede Fraktion 2 mal mit jeweils drei Wiederholungen getestet wurde. Nach 12 Tagen für Test 1 bzw. 14 Tagen für Test 2 wurden die Zellen durch Trypsinierung von der Kulturplatte gelöst und die Zellzahl in den einzelnen Vertiefungen durch Zählung ermittelt.

Die biologische Aktivität der Fraktionen bestimmte sich aus der Differenz aus Zellzahl am Ende und zu Beginn der Kultivierung. Die so ermittelte Differenz entspricht der während der Kultur durch Proliferation erzeugten Zellen, die sich in Abhängigkeit von der biologischen Aktivität der zugesetzten CCM-Fraktion verändert. Biologisch aktive Fraktionen steigern dabei die Proliferation der Zellen im Vergleich zur Negativkontrolle, wobei die Steigerung jedoch maximal den Wert der Positivkontrolle erreicht.

Der Test mit den humanen mesenchymalen Stammzellen wurde analog durchgeführt, es wurden jedoch abweichend 5000 Zellen pro Vertiefung in 24 Loch-Kulturschalen eingesät.

Fraktionen mit einer biologischer Aktivität, die sich positiv auf die Proliferation der Stammzellen auswirkte, wurden anschließend einer Analyse durch MALDI-TOF Massenspektrometrie unterzogen, um die Peptide bzw. Proteine in der Fraktion zu identifizieren, die der biologischen Aktivität der Fraktionen zu Grunde liegen. Dazu wurden die Proteine der entsprechenden Fraktion zuerst mittels 2D-Gelelektrophorese in einem Proteingel aufgetrennt und die Proteine in der ausgeschnittene Proteinbande proteolytisch restringiert. Nach einer Extraktion der entstandenen Peptide aus dem Gel wurden diese massenspektrometrisch charakterisiert und anhand ihrer physikalischen Daten über eine Datenbankrecherche identifiziert.

## Patentansprüche

1. Verfahren zur Differenzierung isolierter und expandierter Stammzellen aus dem Knochenmark in Retina-spezifische Zellen, bei dem man
a) die Stammzellen in einem geeigneten Kulturmedium expandiert;
b) die expandierten Stammzellen in einem Differenzierungsmedium kultiviert, welches Chorioidea-konditioniertes Medium (CCM) und/oder Retinaextrakt (RE) enthält, wobei die Dichte der Stammzellen zwischen 0,5 × 10³ und 2,5 × 10³ Zellen pro cm² beträgt; und
c) die Retina-spezifischen Zellen durch Trennung der Zellen vom Differenzierungsmedium isoliert.

2. Verfahren nach Anspruch 1, **dadurch gekennzeichnet, daß** die Stammzellen aus dem Knochenmark mesenchymale Stammzellen sind.

3. Verfahren nach Anspruch 2, **dadurch gekennzeichnet, daß** die mesenchymalen Stammzellen mindestens zwei Oberflächenantigene ausgewählt aus der Gruppe bestehend aus CD59, CD90, CD105 und MHC I exprimieren.

4. Verfahren nach Anspruch 3, **dadurch gekennzeichnet, daß** die mesenchymalen Stammzellen die Oberflächenantigene CD90 und CD105 exprimieren.

5. Verfahren nach einem der Ansprüche 1 bis 4, **dadurch gekennzeichnet, daß** man für die Differenzierung die adhärent wachsenden mesenchymalen Stammzellen verwendet.

6. Verfahren nach Anspruch 1, **dadurch gekennzeichnet, daß** die Stammzellen aus dem Knochenmark hämatopoetische Stammzellen sind.

7. Verfahren nach Anspruch 6, **dadurch gekennzeichnet, daß** die hämatopoetischen Stammzellen mindestens ein Oberflächenantigen ausgewählt aus der Gruppe bestehend aus CD34 und CD45 exprimieren.

8. Verfahren nach Anspruch 7, **dadurch gekennzeichnet, daß** die hämatopoetischen Stammzellen die Oberflächenantigene CD34 und CD45 exprimieren.

9. Verfahren nach Anspruch 1, **dadurch gekennzeichnet, daß** die Stammzellen aus dem Knochenmark mesenchymale und hämatopoetische Stammzellen sind.

10. Verfahren nach einem der Ansprüche 1 bis 9, **dadurch gekennzeichnet, daß** man die Zellen im Anschluß an Schritt c) in einem flüssigem Medium suspendiert.

11. Verfahren nach Anspruch 10, **dadurch gekennzeichnet, daß** das Medium ausgewählt ist aus der Gruppe bestehend aus RPMI, DMEM, Iscove's Medium und Medium 199 jeweils allein oder als 1:1-Mischungen mit Ham's F12 Nutrient Mixture, sowie Spezialmedien wie Human Endothelial-SFM, START V, Neurobasal/Neurobasal-A-Medium oder deren Supplemente N-2 und B27.

12. Verfahren nach Anspruch 11, **dadurch gekennzeichnet, daß** die suspendierten Zellen nach Zugabe eines Gefrierschutzes und Proteinen zur Stabilisierung empfindlicher biologischer Substanzen tiefgefroren werden.

13. Verfahren nach Anspruch 12, **dadurch gekennzeichnet, daß** dem Medium als Gefrierschutz DMSO oder Methylzellulose zugesetzt wird.

14. Verfahren nach Anspruch 13, **dadurch gekennzeichnet, daß** als Gefrierschutz 10 % DMSO und zur Stabilisierung empfindlicher biologischer Substanzen 10 % Serum oder 10 % Albumin bei serumfreier Kultur zugesetzt werden.

15. Verfahren nach einem der Ansprüche 1 bis 14, **dadurch gekennzeichnet, daß** das Differenzierungsmedium 1 bis 20 % CCM und/oder 0,1 bis 5,0 % RE enthält.

16. Verfahren nach einem der Ansprüche 1 bis 14, **dadurch gekennzeichnet, daß** das Differenzierungsmedium 1 bis 15 % CCM und/oder 0,5 bis 5,0 % RE enthält.

17. Verfahren nach einem der Ansprüche 1 bis 14, **dadurch gekennzeichnet, daß** das Differenzierungsmedium 10 % CCM und/oder 1 % RE enthält.

18. Verfahren nach einem der Ansprüche 1 bis 17, **dadurch gekennzeichnet, daß** man in Schritt b) des Verfahrens die Zellen 3 bis 21 Tage in dem Differenzierungsmedium kultiviert.

19. Verfahren nach Anspruch 18, **dadurch gekennzeichnet, daß** man in Schritt b) des Verfahrens die Zellen 14 bis 21 Tage in dem Differenzierungsmedium kultiviert.

20. Verfahren nach einem der Ansprüche 1 bis 18, **dadurch gekennzeichnet, daß** man in Schritt b) des Verfahrens die Zellen während einer Kulturdauer von 3 bis 14 Tagen andifferenziert.

21. Verfahren nach Anspruch 20, **dadurch gekennzeichnet, daß** man in Schritt b) des Verfahrens die Zellen während einer Kulturdauer von 3 bis 9 Tagen andifferenziert.

22. Verfahren nach Anspruch 20, **dadurch gekennzeichnet, daß** man in Schritt b) des Verfahrens die Zellen während einer Kulturdauer von 5 Tagen andifferenziert.

23. Verfahren nach einem der Ansprüche 1 bis 17, **dadurch gekennzeichnet, daß** man in Schritt b) des Verfahrens die andifferenzieren Stammzellen nach einer Kulturdauer von 3 bis 14 Tagen in einem CCM enthaltendem Differenzierungsmedium für 1 bis 28 Tage in RE enthaltendem Differenzierungsmedium weiter differenziert.

24. Verfahren nach Anspruch 23, **dadurch gekennzeichnet, daß** in Schritt b) des Verfahrens die andifferenzieren Stammzellen nach einer Kulturdauer von 3 bis 5 Tagen in einem CCM enthaltendem Differenzierungsmedium für 1 bis 14 Tage in einem RE enthaltendem Differenzierungsmedium weiter differenziert.

25. Verfahren nach Anspruch 23 oder 24, **dadurch gekennzeichnet, daß** in Schritt b) des Verfahrens die andifferenzierten Stammzellen nach einer Kultur in einem CCM enthaltendem Differenzierungsmedium statt in einem RE enthaltendem Differenzierungsmedium in einem für Neuronenkulturen ausgewiesenen Spezialmedium weiter differenziert werden.

26. Verfahren nach Anspruch 25, **dadurch gekennzeichnet, daß** das zur Weiterdifferenzierung verwendete Spezialmedium Neurobasal oder START V ist.

27. Verfahren nach einem der Ansprüche 1 bis 26, **dadurch gekennzeichnet, daß** die Dichte der Stammzellen in Schritt b) des Verfahrens 2,0 × 10³ Zellen pro cm² beträgt.

28. Verwendung von Chorioidea-konditioniertem Medium (CCM) und/oder Retina-Extrakt (RE) zur Differenzierung von Stammzellen aus dem Knochenmark in Retina-spezifische Zellen.

29. Verwendung nach Anspruch 28, **dadurch gekennzeichnet, daß** die Stammzellen aus dem Knochenmark mesenchymale Stammzellen sind.

30. Verwendung nach Anspruch 28, **dadurch gekennzeichnet, daß** die Stammzellen aus dem Knochenmark hämatopoetische Stammzellen sind.

31. Verwendung nach Anspruch 28, **dadurch gekennzeichnet, daß** die Stammzellen aus dem Knochenmark mesenchymale und hämatopoetische Stammzellen sind.

32. Verwendung nach einem der Ansprüche 28 bis 31, **dadurch gekennzeichnet, daß** das Chorioidea-konditionierte Medium (CCM) einen oder mehrere Wachstumsfaktoren aus der Gruppe bestehend aus Mitgliedern der FGF-Familie, Mitgliedern der Neurotrophin-Familie, Mitgliedern der BMP-Familie, PDGF, EGF, BDNF, CNTF, HGF und NGF enthält.

33. Verwendung nach Anspruch 32, **dadurch gekennzeichnet, daß** das Mitglied der FGF-Familie basischer Fibroblasten-Wachstumsfaktor (bFGF, FGF-2) ist.

34. Verwendung nach Anspruch 32, **dadurch gekennzeichnet, daß** die Mitglieder der Neurotrophin-Familie NT-3 und NT-4 sind.

35. Verwendung nach Anspruch 32, **dadurch gekennzeichnet, daß** das Mitglied der BMP-Familie BMP-4 ist.

36. Verwendung nach Anspruch 32, **dadurch gekennzeichnet, daß** PDGF ausgewählt ist aus der Gruppe bestehend aus PDGF-AA, PDGF-BB und PDGF-AB.

37. Verfahren nach einem der Ansprüche 1 bis 27, **dadurch gekennzeichnet, daß** man die Stammzellen entweder im Anschluß an die Isolierung aus dem Knochenmark, im Verlauf des Differenzierungsverfahrens im Anschluß an die Expansion im Verfahrensschritt a), im Anschluß an die Differenzierung im Verfahrensschritt b) oder die daraus differenzierten Retina-spezifischen Zellen mit einem oder mehreren Fremdgenen transfiziert.

## Claims

1. Method for differentiating isolated and expanded stem cells from the bone marrow into retina specific cells,
wherein
(a) the stem cells are expanded in an appropriate culture medium;
(b) the expanded stem cells are cultivated in a differentiation medium comprising Chorioidea conditioned medium (CCM) and/or retina extract (RE), wherein the density of stem cells is between 0,5 x 10³ and 2.5 x 10³ cells per cm²; and
(c) the retina specific cells are isolated by separation of cells from the differentiation medium.

2. Method of claim 1, wherein the stem cells from the bone marrow are mesenchymal stem cells.

3. Method of claim 2, wherein the mesenchymal stem cells express at least two surface antigens selected from the group consisting of CD59, CD90, CD105 and MHC I.

4. Method of claim 2, wherein the mesenchymal stem cells express the surface antigens CD90 and CD105.

5. Method of any of claims 1 to 4, wherein adherently growing mesenchymal stem cells are used for the differentiation.

6. Method of claim 1, wherein the stem cells from the bone marrow are hematopoietic stem cells.

7. Method of claim 6, wherein the hematopoietic stem cells express at least one surface antigen selected from the group consisting of CD34 and CD45.

8. Method of claim 7, wherein the hematopoietic stem cells express the surface antigen CD34 and CD45.

9. Method of claim 1, wherein stem cells from the bone marrow are mesenchymal and hematopoietic stem cells.

10. Method of any of claims 1 to 9, wherein the cells are suspended in a liquid medium after step c).

11. Method of claim 10, wherein the medium is selected from the group consisting of RPMI, DMEM, Iscove's medium and Medium 199, each alone or as a 1:1 mixture with Ham's F12 nutrient mixture, as well as special media such as Human Endothelial-SFM, START V, Neurobasal/Neurobasal-A-Medium or their supplements N-2 and B27.

12. Method of claim 11, wherein the suspended cells are frozen after addition of freeze protection and proteins for stabilising sensitive biological substances.

13. Method of claim 12, wherein DMSO or methylcellulose are added to the medium as freeze protection.

14. Method of claim 13, wherein 10 % DMSO as freeze protecttion and 10 % serum or with serum free culture 10 % albumin for stabilising sensitive biological substances are added.

15. Method of any of claims 1 to 14, wherein the differenttiation medium comprises 1 to 20 % CCM and/or 0.1 to 5.0 % RE.

16. Method of any of claims 1 to 14, wherein the differenttiation medium comprises 1 to 15 % CCM and/or 0.5 to 5.0 % RE.

17. Method of any of claims 1 to 14, wherein the differenttiation medium comprises 10 % CCM and/or 1% RE.

18. Method of any of claims 1 to 17, wherein in step b) of the method, the cells are a cultivated for 3 to 21 days in the differentiation medium.

19. Method of claim 18, wherein in step b) of the method, the cells are cultivated for 14 to 21 days in the differentiation medium.

20. Method of any of claims 1 to 18, wherein in step b) of the method, the differentiation of cells is started during a culture period of 3 to 14 days.

21. Method of claim 20, wherein in step b) of the method, the differentiation of cells is started during a culture period of 3 to 9 days.

22. Method of claim 20, wherein in step b) of the method, the differentiation of cells is started during a culture period of 5 days.

23. Method of any of the claims 1 to 17, wherein in step b) of the method, the stem cells, having started differentiation after a culture period of 3 to 14 days in a differentiation medium comprising CCM, are further differentiated in a differentiation medium comprising RE for 1 to 28 days.

24. Method of claim 23, wherein in step b) of the method, the stem cells, having started differentiation after a culture period of 3 to 5 days in a differentiation medium comprising CCM, are further differentiated in a differentiation medium comprising RE for 1 to 14 days.

25. Method of claim 23 or 24, wherein in step b) of the method, the stem cells, having started differentiation after culture in a differentiation medium comprising CCM, are further differentiated in a special medium taught for neuron culture instead of a differentiation medium comprising RE.

26. Method of claim 25, wherein the special medium used for further differentiation is Neurobasal or START V.

27. Method of any of claims 1 to 26, wherein the density of stem cells in step b) of the method is 2.0 x 10³ cells per cm².

28. Use of Chorioidea conditioned medium (CCM) and/or retina extract (RE) for the differentiation of stem cells from bone marrow into retina specific cells.

29. Use of claim 28, wherein the stem cells from the bone marrow are mesenchymal stem cells.

30. Method according to claim 28, wherein the stem cells from the bone marrow are hematopoietic stem cells.

31. Method according to claim 28, wherein the stem cells from the bone marrow are mesenchymal and hematopoetic stem cells.

32. Use according to any of claims 28 to 31, wherein the Chorioidea conditioned medium (CCM) comprises one or more growth factors from the group consisting of members of the FGF family, members of the neurotrophin family, members of the BMP family, PDGF, EGF, BDNF, CNTF, HGF and NGF.

33. Use according to claim 32, wherein the member of the FGF family is basic fibroblast growth factor (bFGF, FGF-2).

34. Use according to claim 32, wherein the members of the neurotrophin family are NT-3 and NT-4.

35. Use according to claim 32, wherein the member of the BMP family is BMP-4.

36. Use according to claim 32, wherein PDGF is selected from the group consisting of PDGF-AA, PDGF-BB and PDGF-AB.

37. Method according to any of claims 1 to 27, wherein the stem cells are transfected with one or more external genes either after isolation from the bone marrow, during the differentiation method after the expansion in step a) of the method, after the differentiation in step b) of the method, or the retina specific cells differentiated therefrom.

## Revendications

1. Procédé de différenciation en cellules spécifiques de rétine, de cellules souches isolées et mises en expansion à partir de la moelle osseuse, dans lequel
(a) les cellules souches sont mises en expansion dans un milieu de culture approprié ;
(b) les cellules souches mises en expansion sont cultivées dans un milieu de différentiation comprenant un milieu conditionné choroïde (CCM) et/ou un extrait de rétine (RE) dans lequel la densité des cellules souches est comprise entre 0,5 x 10³ et 2,5 x 10³ cellules par cm² ; et
(c) les cellules spécifiques de rétine sont isolées par séparation des cellules du milieu de différentiation.

2. Procédé selon la revendication 1, dans lequel les cellules souches provenant de la moelle osseuse sont des cellules souches de mésenchyme.

3. Procédé selon la revendication 2, dans lequel les cellules souches de mésenchyme expriment au moins deux antigènes de surface choisis dans le groupe constitué de CD59, CD90, CD105 et MHC I.

4. Procédé selon la revendication 2, dans lequel les cellules souches de mésenchyme expriment les antigènes de surface CD90 et CD105.

5. Procédé selon l'une quelconque des revendications 1 à 4, dans lequel les cellules souches de mésenchyme croissant de façon adhérente sont utilisées pour la différentiation.

6. Procédé selon la revendication 1, dans lequel les cellules souches de la moelle osseuse sont des cellules souches hématopoïétiques.

7. Procédé selon la revendication 6, dans lequel les cellules souches hématopoïétiques expriment au moins un antigène de surface choisi dans le groupe constitué de CD34 et CD45.

8. Procédé selon la revendication 7, dans lequel les cellules souches hématopoïétiques expriment les antigènes de surface CD34 et CD45.

9. Procédé selon la revendication 1, dans lequel les cellules souches de la moelle osseuse sont des cellules souches de mésenchyme et hématopoïétiques.

10. Procédé selon l'une quelconque des revendications 1 à 9, dans lequel les cellules sont resuspendues dans un milieu liquide après l'étape c).

11. Procédé selon la revendication 10, dans lequel le milieu est choisi dans le groupe constitué de RPMI, DMEM, le milieu d'Iscove et le Milieu 199, chacun seul ou en un mélange de proportion 1 :1 avec un mélange nutritif Ham's F12 ainsi qu'un milieu spécial tel qu'un SFM humain endothélial, START V, un milieu Neurobasal/Neurobasal-A ou leurs suppléments N-2 et B27.

12. Procédé selon la revendication 11, dans lequel les cellules suspendues sont congelées après addition d'une protection contre le gel et de protéines pour stabiliser les substances biologiquement sensibles.

13. Procédé selon la revendication 12, dans lequel le DMSO ou la méthylcellulose sont ajoutés au milieu en tant que protection contre le gel.

14. Procédé selon la revendication 13, dans lequel 10% de DMSO en tant que protection contre le gel et 10% de sérum ou 10% d'albumine sans sérum sont ajoutés pour stabiliser les substances biologique sensibles.

15. Procédé selon l'une quelconque des revendications 1 à 14, dans lequel le milieu de différentiation comprend de 1 à 20% de CCM et/ou de 0,1 à 5% de RE.

16. Procédé selon l'une quelconque des revendications 1 à 14, dans lequel le milieu de différentiation comprend de 1 à 15% de CCM et/ou de 0,5 à 5% de RE.

17. Procédé selon l'une quelconque des revendications 1 à 14, dans lequel le milieu de différentiation comprend 10% de CCM et/ou 1 % de RE.

18. Procédé selon l'une quelconque des revendications 1 à 17, dans lequel dans l'étape b) du procédé, les cellules sont cultivées pendant 3 à 21 jours dans le milieu de différentiation.

19. Procédé selon la revendication 18, dans lequel dans l'étape b) du procédé, les cellules sont cultivées pendant 14 à 21 jours dans le milieu de différentiation.

20. Procédé selon l'une quelconque des revendications 1 à 18, dans lequel dans l'étape b) du procédé, la différentiation des cellules est commencée pendant une période de culture de 3 à 14 jours.

21. Procédé selon la revendication 20, dans lequel dans l'étape b) du procédé, la différentiation des cellules est commencée pendant une période de culture de 3 à 9 jours.

22. Procédé selon la revendication 20, dans lequel dans l'étape b) du procédé, la différentiation des cellules est commencée pendant une période de culture de 5 jours.

23. Procédé selon l'une quelconque des revendications 1 à 17, dans lequel dans l'étape b) du procédé, les cellules souches, ayant démarré une différentiation après une période de culture de 3 à 14 jours dans un milieu de différentiation comprenant du CCM, sont par la suite différenciées dans un milieu de différentiation comprenant RE pendant 1 à 28 jours.

24. Procédé selon la revendication 23, dans lequel dans l'étape b) du procédé, les cellules souches, ayant démarré une différentiation après une période de culture de 3 à 5 jours dans un milieu de différentiation comprenant du CCM, sont par la suite différenciées dans un milieu de différentiation comprenant RE pendant 1 à 14 jours.

25. Procédé selon la revendication 23 ou 24, dans lequel dans l'étape b) du procédé, les cellules souches, ayant démarré une différentiation après une culture dans un milieu de différentiation comprenant du CCM, sont par la suite différenciées dans un milieu spécial dit pour culture de neurones à la place d'un milieu de différentiation comprenant RE.

26. Procédé selon la revendication 25, dans lequel le milieu spécial utilisé pour la différentiation est le Neurobasal ou le START V.

27. Procédé selon l'une quelconque des revendications 1 à 26, dans lequel la densité des cellules souches dans l'étape b) du procédé est de 2 x 10³ cellules par cm².

28. Utilisation d'un milieu conditionné choroïde (CCM) et/ou d'un extrait de rétine (RE) pour la différentiation des cellules souches de moelle osseuse en cellules spécifiques de rétine.

29. Utilisation selon la revendication 28, dans laquelle les cellules souches provenant de la moelle osseuse sont des cellules souches de mésenchyme.

30. Procédé selon la revendication 28, dans lequel les cellules souches provenant de la moelle osseuse sont des cellules souches hématopoïétiques.

31. Procédé selon la revendication 28, dans lequel les cellules souches provenant de la moelle osseuse sont des cellules souches de mésenchyme et hématopoïétiques.

32. Utilisation selon l'une quelconque des revendications 28 à 31, dans laquelle le milieu conditionné choroïde (CCM) comprend un ou plusieurs facteurs de croissance dans le groupe constitué des membres de la famille FGF, des membres de la famille des neurotrophines, des membres de la famille des BMP, PDGF, EGF, BDNF, CNTF, HGF et NGF.

33. Utilisation selon la revendication 32, dans laquelle le membre de la famille FGF est le facteur de croissance basique des fibroblastes (bFGF, FGF-2).

34. Utilisation selon la revendication 32, dans laquelle les membres de la famille des neurotrophines sont NT-3 et NT-4.

35. Utilisation selon la revendication 32, dans laquelle le membre de la famille des BMP est BMP-4.

36. Utilisation selon la revendication 32, dans laquelle le PDGF est choisi dans le groupe constitué de PDGF-AA, PDGF-BB et PDGF-AB.

37. Procédé selon l'une quelconque des revendications 1 à 27, dans lequel les cellules souches sont transfectées avec un ou plusieurs gènes externes soit après l'isolement à partir de la moelle osseuse, durant le procédé de différentiation après l'expansion à l'étape a) du procédé, après la différentiation à l'étape b) du procédé, ou les cellules spécifiques de rétine différenciées qui en découlent.
